# EUROPÄISCHE PATENTANMELDUNG

(11) **EP 3 424 970 A1**
(43) Veröffentlichungstag der Anmeldung: **09.01.2019**
(21) Anmeldenummer: 17179291.4
(22) Anmeldetag: 03.07.2017
(51) Int. Cl.: C08G 18/67, C08G 18/76, C08G 18/24, C08G 18/32, C08L 75/16, C08G 18/10

(54) **VERZWEIGTE URETHANMETHACRYLAT-VERBINDUNGEN UND DEREN VERWENDUNG**

(71) Anmelder: HILTI Aktiengesellschaft, 9494 Schaan (LI)
(72) Erfinder: Gnaß, Beate, 86368 Gersthofen (DE); Bunzen, Jens, 86159 Augsburg (DE); Nickerl, Georg, 86911 Dießen am Ammersee (DE)
(74) Vertreter: Hilti Aktiengesellschaft Corporate Intellectual Property

(57) **Zusammenfassung**

Es werden verzweigte Urethanmethacrylat-Verbindungen als Backbone-Harze und deren Verwendung zur Erhöhung der Leistungsfähigkeit einer Befestigungsmasse beschrieben. Des Weiteren wird die Verwendung diese Verbindungen enthaltenden Reaktivharze und Reaktivharzkomponenten zur chemischen Befestigung beschrieben.

## Beschreibung

Die Erfindung betrifft verzweigte Urethanmethacrylat-Verbindungen als Backbone-Harze, deren Verwendung in Reaktivharzen, insbesondere zur Erhöhung der Leistungsfähigkeit solcher Verbindungen enthaltenden Reaktivharzen und damit von daraus hergestellten Reaktivharzkomponenten. Des Weiteren betrifft die Erfindung die Verwendung der Reaktivharze und der Reaktivharzkomponenten zu baulichen Zwecken, insbesondere zur chemischen Befestigung.

Die derzeit eingesetzten radikalisch härtbaren Befestigungsmassen basieren auf linearen ungesättigten Polyestern, Vinylesterurethanharzen und Epoxyacrylaten. Es handelt sich dabei meist um Zweikomponenten-Reaktivharz-Systeme, wobei eine Komponente das Harz (sogenannte Komponente (A)) und die andere Komponente (Komponente (B)) das Härtungsmittel enthält. Weitere Bestandteile wie anorganische Füllmittel und Additive, Beschleuniger, Stabilisatoren und Reaktivverdünner können in der einen und/oder der anderen Komponente enthalten sein. Durch Vermischen der beiden Komponenten wird die Härtung der gemischten Komponenten in Gang setzt. Bei Verwendung der Befestigungsmassen zur Befestigung von Verankerungselementen in Bohrlöchern erfolgt die Härtung in den Bohrlöchern.

Eine solche Befestigungsmasse ist beispielsweise aus der DE 3940138 A1 bekannt. Diese beschreibt Befestigungsmassen auf Basis von cycloaliphatische Gruppen tragenden Monomeren, die zusätzlich ungesättigte Polyester- oder Vinylesterharze enthalten können.

Nachteilig daran und an den bisher in chemischen Befestigungsmassen üblicherweise eingesetzten Harzen ist, dass es sich im Wesentliche um lineare Harze handelt, die lineare Polymerketten geben. Hierdurch ist allerdings die innere Festigkeit und damit die Leistungsfähigkeit eines Mörtels beschränkt.

Daher werden häufig tri- oder höherfunktionelle Reaktivverdünner den Befestigungsmassen zugegeben, damit diese als zusätzliche Vernetzer dienen können, um eine höhere Quervernetzung der Polymerketten zu erreichen. Dies bedeutet allerdings, dass der Anteil an Reaktivverdünner in den Befestigungsmassen zunimmt, was letztendlich zu einer Verringerung des Harzanteils in der Masse führt. Nicht selten beträgt der Anteil an Reaktivverdünnern mindestens 50%, bezogen auf das Reaktivharz.

Die Verwendung von hohen Anteilen an Reaktivverdünnern führt allerdings auch zu einigen Nachteilen, die sich vor allem bei der Anwendung der Befestigungsmasse zur Befestigung von Verankerungsmittel in Bohrlöchern bemerkbar machen.

Ein erheblicher Nachteil ist, dass die Verringerung des Anteils an hochviskosem Harz, welches für die Leistungsfähigkeit der Masse wesentlich ist, die Leistungsfähigkeit der ausgehärteten Befestigungsmasse negativ beeinflusst.

Ein weiterer Nachteil ist ein größerer Schrumpf der Befestigungsmasse nach dem Aushärten, was zusätzlich die Leistungsfähigkeit der ausgehärteten Befestigungsmasse negativ beeinflussen kann. Dies wird darauf zurückgeführt, dass der Kontakt zwischen der ausgehärteten Befestigungsmasse und den beim Erstellen des Bohrlochs in der Bohrlochwandung gebildeten Hinterschnitten, die insbesondere bei der Verwendung von Schlagbohrmaschinen auftreten, deutlich verringert wird. Dies verhindert meist auch die Anwendung der Befestigungsmassen auf Basis radikalisch härtbarer Verbindungen in diamantgebohrten Löchern.

Ein weiterer Nachteil ist, dass sich je nach Art des Reaktivverdünners der Anteil an flüchtigen organischen Verbindungen (VOC; Volatile Organic Compounds) in den Massen erhöhen kann. Dies kann zu Ausdünstungen aus der Befestigungsmasse und/oder der Kartusche führen und gegebenenfalls zu einem daraus resultierenden Leistungsabfall der ausgehärteten Befestigungsmasse. Einige dieser Verbindungen können zudem auch gesundheitsgefährdend sein und/oder sind daher kennzeichnungspflichtig.

Die Anzahl an einsetzbaren Reaktivverdünner ist zudem gering, da es derzeit nur wenige verfügbare Reaktivverdünner auf dem Markt gibt. Die verfügbaren Reaktivverdünner tragen neben den radikalisch härtbaren funktionellen Gruppen keine oder nur eine sehr eingeschränkte Auswahl an anderen funktionellen Gruppen und haben daher oft nur wenig Einfluss auf die Eigenschaft der ausgehärteten Befestigungsmasse. Dies führt dazu, dass die Befestigungsmassen meist für bestimmte Anwendungen entwickelt werden, wie etwa bestimmte Temperaturbereiche, oder zur Anwendung in bestimmten Untergründen. Dies spricht für einen immensen Entwicklungsaufwand, um neue und breitere Anwendungen mit den Befestigungsmassen adressieren zu können.

Bisher werden Spezialprodukte, deren Formulierungen auf die speziellen Anwendungstemperaturen angepasst sind, hergestellt. Es gibt zwar Produkte, die für einen breiten Temperaturbereich gedacht sind, die jedoch über den gesamten Bereich gleiche Eigenschaften aufweisen. Gerade in den Randbereichen, d.h. bei niedrigen und bei hohen Temperaturen, muss entweder mit Beeinträchtigungen bei der Verarbeitbarkeit, bei der Aushärtung der Masse, oder bei den Eigenschaften der ausgehärteten Masse gerechnet werden. Es ist keine Befestigungsmasse bekannt, die einen sehr großen Temperaturbereich abdeckt, ohne Einbußen in den Randbereichen hinnehmen zu müssen.

Es besteht daher ein Bedarf an Befestigungsmassen deren Leistungsfähigkeit und Eigenschaften nicht durch den Einsatz von Reaktivverdünnern, sondern durch den Harzbestandteil beeinflusst werden kann.

Eine Aufgabe der vorliegenden Erfindung ist die Beeinflussung der Eigenschaften eines Reaktivharz-Masterbatches sowie eines daraus hergestellten Reaktivharzes, die allein auf die Struktur des Backbone-Harz zurückzuführen ist, nicht jedoch auf die Präsenz von zusätzlichen Verbindungen, wie z. B. Reaktivverdünner oder Additive. Hauptsächlich ist es Aufgabe der vorliegenden Erfindung die Eigenschaften eines Zwei- oder Mehrkomponenten-Reaktivharz-Systems mittels des enthaltenden Backbone-Harzes zu steuern.

Eine noch weitere Aufgabe der vorliegenden Erfindung ist die Bereitstellung einer Befestigungsmasse, welche hochgradig gesundheitsgefährdende Inhaltsstoffe in der Reaktivharz-Komponente vermeidet und gegebenenfalls auch kennzeichnungsfrei ist. Insbesondere ist es Aufgabe, den Anteil an Reaktivverdünnern in Reaktivharzen für die chemische Befestigung zu verringern, ohne auf deren Funktion bzw. Funktionen und positiven Auswirkungen auf die ausgehärtete Befestigungsmasse verzichten zu müssen.

Eine noch weitere Aufgabe der vorliegenden Erfindung ist die Bereitstellung einer Befestigungsmasse, welche sich durch gute Verarbeitbarkeit, Aushärteverhalten und durch geringen Schrumpf über eine große Temperaturspanne auszeichnet und gleichzeitig höhere Lastwerte der ausgehärteten Befestigungsmasse aufweist als herkömmliche Massen.

Diese Aufgaben werden durch die Verbindungen nach Anspruch 1 und deren Verwendung nach den Ansprüchen 8 und 9, das Reaktivharz nach Anspruch 10 und die Reaktivharzkomponente nach Anspruch 11 gelöst.

Überraschender Weise wurde gefunden, dass durch die Verwendung bestimmter verzweigter Urethanmethacrylat-Verbindungen als Backbone-Harz die Lastwerte einer ausgehärteten Masse gesteigert werden konnten.

Zum besseren Verständnis der Erfindung werden die folgenden Erläuterungen zum Herstellungsverfahren für ein Reaktivharz und zur hierin verwendeten Terminologie als sinnvoll erachtet.

Das Herstellungsverfahren für ein Reaktivharz verläuft, hier erläutert am Beispiel eines MDI-basierten Urethanmethacrylates, typischerweise wie folgt:

### 1. Herstellung Backbone-Harz/Reaktivharz-Masterbatch

Methandiphenyldiisocyanat (MDI), Hydroxypropylmethacrylat (HPMA) und Trimethylolpropan (TMP) werden in Anwesenheit eines Katalysators und eines Inhibitors (dient dazu, das durch die Polymerisation gebildete Backbone-Harz zu stabilisieren, häufig auch Stabilisator oder Prozessstabilisator genannt) zur Reaktion gebracht. Hierbei entsteht das Backbone-Harz.

Das nach Ende der Reaktion erhaltene Reaktionsgemisch wird als Reaktivharz-Masterbatch bezeichnet. Dieses wird nicht weiter aufgearbeitet, d.h. das Backbone-Harz wird nicht isoliert.

### 2. Herstellung Reaktivharz

Zu dem Reaktivharz-Masterbatch werden nach Abschluss der Reaktion zum Backbone-Harz ein Beschleuniger-Inhibitor-System, d.h. eine Kombination aus einem oder mehreren zusätzlichen Inhibitoren und einem oder mehreren Beschleunigern sowie gegebenenfalls ein Reaktivverdünner gegeben.

Hierdurch erhält man das Reaktivharz.

Das Beschleuniger-Inhibitor-System dient dazu, die Reaktivität des Reaktiv-Harzes einzustellen, also den Zeitpunkt einzustellen, bis zu dem das Reaktiv-Harz nach Zugabe eines Initiators noch nicht vollständig ausgehärtet ist und bis zu welchem Zeitpunkt daher eine mit dem Reaktivharz angemischte Dübelmasse nach dem Mischen mit dem Initiator verarbeitbar bleibt.

Der Inhibitor in dem Beschleuniger-Inhibitor-System kann gleich dem Inhibitor bei der Herstellung des Backbone-Harzes sein, wenn dieser auch dazu geeignet ist, die Reaktivität einzustellen, oder ein anderer Inhibitor sein, wenn er nicht beide Funktionen aufweist. 4-Hydroxy-2,2,6,6-tetramethyl-piperidinyl-1-oxyl (TEMPOL) etwa kann als Stabilisator und als Inhibitor zu Einstellung der Reaktivität eingesetzt werden.

### 3. Herstellung Reaktivharzkomponente

Für die Verwendung des Reaktivharzes für bauliche Zwecke, insbesondere für die chemische Befestigung, werden nach der Herstellung des Reaktivharzes ein oder mehrere anorganische Zuschlagstoffe, wie Additive und/oder Füllstoffe, gegeben.

Hierdurch wird die Reaktivharzkomponente erhalten.

Im Sinne der Erfindung bedeuten die verwendeten Begriffe:
- *"Backbone-Harz"* ein üblicherweise festes oder hochviskoses radikalisch polymerisierbares Harz, welches durch Polymerisation (z.B. nach Zugabe eines Initiators in Gegenwart eines Beschleunigers) härtet und in der Regel ohne Reaktivverdünner und ohne weiterer Reinigung vorliegt und damit Verunreinigung enthalten kann;
- *"Reaktivharz-Masterbatch"* das Reaktionsprodukt der Reaktion zur Herstellung des Backbone-Harzes, also eine Mischung aus Backbone-Harz, Reaktivverdünner und gegebenenfalls weiteren Bestandteilen der Reaktionsmischung;
- *"Reaktivharz"* eine Mischung aus Reaktivharz-Masterbatch, mindestens einem Beschleuniger und mindestens einem Inhibitor (auch als Beschleuniger-Inhibitor-System bezeichnet), mindestens einem Reaktivverdünner und gegebenenfalls weiteren Additiven; das Reaktivharz ist typischerweise flüssig oder viskos und kann zu einer Reaktivharzkomponente weiterverarbeitet werden; hierin wird das Reaktivharz auch *als "Harzmischung"* bezeichnet;
- *"Inhibitor"* einen Stoff, der eine unerwünschte radikalische Polymerisation während der Synthese oder der Lagerung eines Harzes oder einer Harz-haltigen Zusammensetzung unterdrückt (diese Stoffe werden in Fachkreisen auch als *"Stabilisator"* bezeichnet) bzw. der eine radikalische Polymerisation eines Harzes nach Zugabe eines Initiators (üblicherweise in Verbindung mit einem Beschleuniger) zeitlich verzögert (diese Stoffe werden in Fachkreisen auch als *"Inhibitor"* bezeichnet - die jeweilige Bedeutung des Begriffes erschließt sich aus dem Kontext);
- *"Beschleuniger"* ein Reagenz, welches mit dem Initiator eine Reaktion eingeht, so dass bereits bei niedrigen Temperaturen durch den Initiator größere Mengen an Radikalen erzeugt werden, oder welches die Zerfallsreaktion des Initiators katalysiert;
- *"Reaktivverdünner"* flüssige oder niedrigviskose Monomere und Backbone-Harze, welche andere Backbone-Harze oder den Reaktivharz-Masterbatch verdünnen und dadurch die zu deren Applikation notwendige Viskosität verleihen, zur Reaktion mit dem Backbone-Harz befähigte funktionelle Gruppen enthalten und bei der Polymerisation (Härtung) zum überwiegenden Teil Bestandteil der gehärteten Masse (z.B. des Mörtels) werden; Reaktivverdünner werden auch co-polymerisierbares Monomer genannt;
- *"Reaktivharzkomponente"* eine flüssige oder viskose Mischung aus Reaktivharz und Füllstoffen und optional weiteren Komponenten, z.B. Additiven; typischerweise ist die Reaktivharzkomponente eine der beiden Komponenten eines Zweikomponenten-Reaktivharz-Systems zur chemischen Befestigung;
- *"Initiator"* einen Stoff, der (üblicherweise in Kombination mit einem Beschleuniger) reaktionsinitierende Radikale bildet;
- *"Härterkomponente"* eine Zusammensetzung, die einen Initiator für die Polymerisation eines Backbone-Harzes enthält; die Härterkomponente kann fest oder flüssig sein und neben dem Initiator ein Lösungsmittel sowie Füllstoffe und/oder Additive enthalten; typischerweise ist die Härterkomponente neben der Reaktivharzkomponente die andere der beiden Komponenten eines Zweikomponenten-Reaktivharz-Systems zur chemischen Befestigung;
- *"Mörtelmasse*/*Befestigungsmasse"* die Zusammensetzung, die durch das Mischen der Reaktivharzkomponente mit der Härterkomponente erhalten wird und als solche direkt zur chemischen Befestigung verwendet werden kann;
- *"Reaktivharz-System"* allgemein ein System, das voneinander getrennt gelagerte Komponenten umfasst, so dass eine Härtung des in einer Komponente enthaltenen Backbone-Harzes erst nach dem Mischen der Komponenten erfolgt;
- *"Zweikomponenten-System"* bzw. *"Zweikomponenten-Reaktivharz-System"* ein Reaktivharz-System, das zwei voneinander getrennt gelagerte Komponenten, eine Reaktivharzkomponente (A) und eine Härterkomponente (B), umfasst, so dass eine Härtung des in der Reaktivharzkomponente enthaltenen Backbone-Harzes erst nach dem Mischen der beiden Komponenten erfolgt;
- *"Mehrkomponenten-System"* bzw. *"Mehrkomponenten-Reaktivharz-System"* ein Reaktivharz-System, das mehrere voneinander getrennt gelagerte Komponenten umfasst, unter anderem eine Reaktivharzkomponente (A) und eine Härterkomponente (B), so dass eine Härtung des in der Reaktivharzkomponente enthaltenen Backbone-Harzes erst nach dem Mischen aller Komponenten erfolgt;
- *"bauliche Zwecke"* jegliche Anwendung zur Erstellung und Instandhaltung bzw. - setzung von Bauteilen und Bauwerken, als Polymerbeton, als Beschichtungsmasse auf Kunstharzbasis oder als kalthärtende Straßenmarkierung; insbesondere die Verstärkung von Bauteilen und Bauwerken, beispielsweise Wände, Decken oder Böden, die Befestigung von Bauteilen, wie Platten oder Blöcken, z. B. aus Stein, Glas oder Kunststoff, an Bauteilen oder Bauwerken, etwa durch Kleben (bauliches Kleben) und ganz besonders die chemische Befestigung von Verankerungsmitteln, wie Ankerstangen, Bolzen oder dergleichen in Aussparungen, wie Bohrlöchern;
- *"chemische Befestigung"* eine (stoff- und/oder formschlüssige) Befestigung von Verankerungsmitteln, wie Ankerstangen, Bolzen, Bewehrungseisen, Schrauben oder dergleichen in Aussparungen, wie Bohrlöchern, insbesondere in Bohrlöchern in verschiedenen Untergründen, insbesondere mineralischen Untergründen, wie solche auf der Grundlage von Beton, Porenbeton, Ziegelwerk, Kalksandstein, Sandstein, Naturstein, Glas und dergleichen, und metallischen Untergründen, wie solche aus Stahl;
- *"aromatische Kohlenwasserstoffgruppe",* eine cyclische, planare Kohlenwasserstoffgruppe mit aromatischem System, die aufgrund ihres delokalisierten Elektronensystems energetisch günstiger als ihre nicht aromatischen Mesomere und deshalb chemisch stabiler sind (PAC, 1995, 67, 1307; Glossary of class names of organic compounds and reactivity intermediates based on structure (IUPAC Recommendations 1995) page 1319);
- *"aromatisches Diisocyanat",* dass die beiden Isocyanatgruppen direkt an ein aromatisches Kohlenwasserstoffgerüst gebunden sind;
- *"(Meth)acryl...*/*...(meth)acryl...",* dass sowohl die "Methacryl.../...methacryl..."- als auch die "Acryl.../...acryl..."-Verbindungen gemeint sein sollen; bevorzugt sind in der vorliegenden Erfindung "Methacryl.../...methacryl... "-Verbindungen gemeint;
- "*ein*", *"eine", "einer"* als Artikel vor einer chemischen Verbindungsklasse, z.B. vor dem Wort "Urethanmethacrylat", dass mindestens eine, d.h., eine oder mehrere unter diese chemische Verbindungsklasse fallende Verbindungen, z.B. verschiedene Urethanmethacrylate, gemeint sein können. In einer bevorzugten Ausführungsform ist mit diesem Artikel nur eine einzelne Verbindung gemeint;
- *"mindestens ein", "mindestens eine", "mindestens einer"* zahlenmäßig *"ein oder mehrere".* In einer bevorzugten Ausführungsform ist mit diesem Begriff zahlenmäßig *"ein", "eine", "einer"* gemeint;
- *"enthalten" und "umfassen"*, dass neben den genannten Bestandteilen noch weitere vorhanden sein können. Diese Begriffe sind einschließlich gemeint und umfassen daher auch *"bestehen aus". "Bestehen aus"* ist abschließend gemeint und bedeutet, dass keine weiteren Bestandteile vorhanden sein können. In einer bevorzugten Ausführungsform bedeuten die Begriffe *"enthalten" und "umfassen"* den Begriff *"bestehen aus*";
- *"etwa"* oder *"circa"* vor einem Zahlenwert einen Bereich von ± 5% dieses Wertes, bevorzugt ± 2% dieses Wertes, bevorzugter ± 1% dieses Wertes, besonders bevorzugt ± 0% dieses Wertes (also genau diesen Wert);
- ein durch Zahlen begrenzter Bereich, dass die beiden Eckwerte und jeder Wert innerhalb dieses Bereichs einzeln offenbart sind.

Alle in diesem Text genannten Normen (z.B. DIN-Normen) wurden in der zum Anmeldetag dieser Anmeldung aktuellen Ausgabe verwendet.

Ein erster Gegenstand der Erfindung ist eine Verbindung der allgemeinen Formel (I) 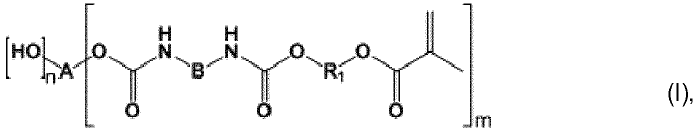 worin
B eine aromatische Kohlenwasserstoffgruppe ist,
A eine lineare oder verzweigte aliphatische C₃-C₁₀-Alkylengruppe ist,
jedes R₁ jeweils unabhängig voneinander eine verzweigte oder lineare aliphatische C₁-C₁₅-Alkylengruppe ist,
n eine ganze Zahl größer oder gleich 0 ist, und
m eine ganze Zahl größer oder gleich 3 ist.

Ein zweiter Gegenstand ist deren Verwendung zur Herstellung eines Reaktivharzes oder einer Reaktivharzkomponente für bauliche Zwecke. Ein dritter Gegenstand ist deren Verwendung zur Erhöhung der Verbundspannung einer ausgehärteten Befestigungsmasse. Ein vierter Gegenstand ist ein Reaktivharz umfassend eine Verbindung der allgemeinen Formel (I), einen Inhibitor, einen Beschleuniger und gegebenenfalls einen Reaktivverdünner. Ein fünfter Gegentand ist eine Reaktivharzkomponente für ein Reaktivharz-System umfassend das Reaktivharz. Ein sechster Gegenstand ist ein Reaktivharz-System mit der Reaktivharzkomponente und einer Härterkomponente, die einen Initiator enthält. Ein siebter Gegenstand ist die Verwendung des Reaktivharzes oder des Reaktivharz-Systems für bauliche Zwecke, insbesondere für die chemische Befestigung.

Erfindungsgemäß ist die verzweigte Urethanmethacrylat-Verbindung eine Verbindung der allgemeinen Formel (I) 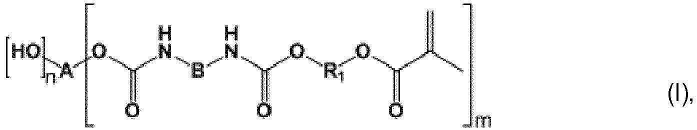 worin
B eine aromatische Kohlenwasserstoffgruppe ist,
A eine lineare oder verzweigte aliphatische C₃-C₁₀-Alkylengruppe ist,
jedes R₁ jeweils unabhängig voneinander eine verzweigte oder lineare aliphatische C₁-C₁₅-Alkylengruppe ist,
n eine ganze Zahl größer oder gleich 0 ist, und
m eine ganze Zahl größer oder gleich 3 ist.

In einer bevorzugten Ausführungsform der Erfindung ist die verzweigte Urethanmethactrylat-Verbindung eine Verbindung der allgemeinen Formel (II) 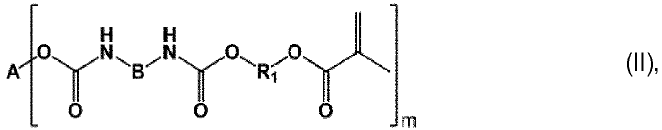 worin
B eine aromatische Kohlenwasserstoffgruppe ist,
A eine lineare oder verzweigte aliphatische C₃-C₁₀-Alkylengruppe ist,
jedes R₁ jeweils unabhängig voneinander eine verzweigte oder lineare aliphatische C₁-C₁₅-Alkylengruppe ist, und
m eine ganze Zahl größer oder gleich 3 ist.

Die aromatische Kohlenwasserstoffgruppe B ist zweiwertig und hat bevorzugt 6 bis 20 Kohlenstoffatome und weiter bevorzugt 6 bis 14 Kohlenstoffatome. Die aromatische Kohlenwasserstoffgruppe kann substituiert sein, insbesondere durch Alkylreste, worunter Alkylreste mit einem bis vier Kohlenstoffatomen bevorzugt sind.

In einer Ausführungsform enthält die aromatische Kohlenwasserstoffgruppe einen Benzolring, der substituiert sein kann.

In einer alternativen Ausführungsform enthält die aromatische Kohlenwasserstoffgruppe zwei kondensierte Benzolringe oder zwei Benzolringe, die über eine Alkylengruppe, wie eine Methylen oder Ethylengruppe verbrückt sind. Sowohl die Benzolringe als auch die Alkylenbrücke kann substitutiert sein, bevorzugt mit Alkylgruppen.

Die aromatische Kohlenwasserstoffgruppe ist von aromatischen Diisocyanaten abgeleitet, wobei "aromatisches Diisocyanat" bedeutet, dass die beiden Isocyanatgruppen direkt an ein aromatisches Kohlenwasserstoffgerüst gebunden sind.

Geeignete aromatische Kohlenwasserstoffgruppen sind zweiwertige Gruppen, wie sie durch Entfernung der Isocyanatgruppen aus einem aromatischen Diisocyanat erhalten werden, beispielsweise eine zweiwertige Phenylengruppe aus einem Benzoldiisocyanat, eine Methylphenylengruppe aus einem Toluoldiisocyanat (TDI) oder eine Ethylphenylengruppe aus einem Ethylbenzoldiisocyanat, eine zweiwertige Methandiphenylengruppe aus einem Methandiphenyldiisocyanat (MDI) oder eine zweiwertige Naphthylgruppe aus einem Naphthalendiisocyanat (NDI).

Besonders bevorzugt ist B abgeleitet von 1,3-Diisocyanatobenzol, 1,4-Diisocyanatobenzol, 2,4-Diisocyanatotoluol, 2,6-Diisocyanatotoluol, 2,4'-Diphenylmethandiisocyanat, 4,4'-Diphenylmethandiisocyanat oder 1,5-Diisocyanatonaphthalin.

R₁ ist jeweils unabhängig voneinander eine verzweigte oder lineare aliphatische C₁-C₁₅-Alkylengruppe, die substituiert sein kann. R₁ ist von Hydroxyalkylmethacrylaten abgeleitet und umfasst zweiwertige Alkylengruppen, wie sie durch Entfernung der Hydroxylgruppen und der Methyacrylatestergruppe erhalten werden.

In einer Ausführungsform ist die Alkylengruppe R₁ zweiwertig.

In einer alternativen Ausführungsform kann sie aber auch drei- oder höherwertig sein, so dass die Verbindung der Formel (I) auch mehr als zwei Methacrylatgruppen aufweisen kann, auch wenn dies nicht direkt aus der Formel (I) oder Formel (II) ersichtlich ist.

Bevorzugt ist die Alkylengruppe R₁ eine zweiwertige lineare oder verzweigte C₁-C₁₅-Alkylengruppe, bevorzugt C₁-C₆-Alkylengruppe und besonders bevorzugt eine C₁-C₄-Alkylengruppe. Dazu zählen insbesondere die Methylen-, Ethylen-, Propylen-, *i*-Propylen-, *n*-Butylen-, 2-Butylen-, *sec.*-Butylen-, *tert*.-Butylen-, *n*-Pentylen-, 2-Pentylen-, 2-Methylbutylen-, 3-Methylbutylen-, 1,2-Dimethylpropylen-, 1,1-Dimethylpropylen-, 2,2-Dimethylpropylen-, 1-Ethylpropylen-, *n*-Hexylen-, 2-Hexylen-, 2-Methylpentylen-, 3-Methylpentylen-, 4-Methylpentylen-, 1,2-Dimethylbutylen-, 1,3-Dimethylbutylen-, 2,3-Dimethylbutylen-, 1,1-Dimethylbutylen-, 2,2-Dimethylbutylen-, 3,3-Dimethylbutylen-, 1,1,2-Trimethylpropylen-, 1,2,2-Trimethylpropylen-, 1-Ethylbutylen-, 2-Ethylbutylen-, 1-Ethyl-2-methylpropylen-, *n-*Heptylen-, 2-Heptylen-, 3-Heptylen-, 2-Ethylpentylen-, 1-Propylbutylen- oder die Octylengruppe, worunter die Ethylen-, Propylen- und Isopropylengruppe weiter bevorzugt sind. In einer besonders bevorzugten Ausführungsform der vorliegenden Erfindung sind die beiden R₁-Gruppen identisch und eine Ethylen-, Propylen- oder *i-*Propylengruppe.

Die aliphatische C₃-C₁₀-Alkylengruppe A dient als Gerüst, an welchem die Urethanmethacrylat-Gruppen hängen, und ist eine drei- oder höherwertige C₃-C₁₀-Alkylengruppe, die linear oder verzweigt ist.

Geeignete lineare oder verzweigte aliphatische C₃-C₁₀-Alkylengruppen sind drei- oder höherwertige, bevorzugt drei- oder vierwertige Gruppen, wie sie durch Entfernung der Hydroxylgruppen aus einem tri- oder höherfunktionellen, bevorzugt tri- oder tetrafunktionellen Alkohol erhalten werden. Entsprechend geeignete Alkohole sind beispielsweise Glycerin, Trimethylolmethan, Trimethylolethan, Trimethylolpropan, Trimethylolbutan, 1,2,4-Butantriol, 1,2,3-Hexantriol, 1,2,4-Hexantriol, Pentaerythrit, Diglycerin, Triglycerin, Bis(trimethylolpropan), Zucker, wie zum Beispiel Glucose, Zuckerderivate, wie z.B. Sorbit, Mannit, Diglycerol, Threit, Erythrit, Adonit (Ribit), Arabit (Lyxit), Xylit, Dulcit (Galactit), Maltit, Isomalt, oder Polyesterol.

Hierdurch wird erreicht, dass mehr als zwei radikalisch härtbare Gruppen, nämliche die Methacrylatgruppen, pro Verbindung enthalten sind, so dass die Verbindungen bei der Polymerisation als sogenannte Vernetzer dienen. Die Verwendung dieser Verbindungen kann aufgrund der weiteren Methacrylatgruppe zu einer Quervernetzung der gebildeten Polymerketten führen, so sich ein vernetztes Polymernetzwerk ausbilden kann.

Dementsprechend entspricht m zusammen mit n (m + n) der Wertigkeit des zur Herstellung der erfindungsgemäßen Verbindung verwendeten Alkohols. Folglich ist m = 3 wenn ein trifunktioneller Alkohol und m + n = 3 oder 4 wenn ein tetrafunktioneller Alkohol verwendet wird.

Durch die Steuerung der Reaktionsbedingungen ist es möglich, dass nicht alle Hydroxylgruppen mit einer Isocyanatgruppe reagieren, so dass in der erhaltenen Verbindung noch freie Hydroxylgruppen vorhanden sind. Diese Verbindungen sind ebenfalls vom Umfang der Erfindung umfasst.

Hieraus resultieren Verbindungen der allgemeinen Formel (I), in denen n > 0 ist.

Werden alle Hydroxylgruppen umgesetzt, so dass in der erhaltenen Verbindung keine freien Hydroxylgruppen mehr vorhanden sind, entspricht m alleine (n = 0) der Wertigkeit des zur Herstellung der erfindungsgemäßen Verbindung verwendeten Alkohols. Folglich ist m = 3 wenn ein trifunktioneller Alkohol und m = 4 wenn ein tetrafunktioneller Alkohol verwendet wird.

Hieraus resultieren Verbindungen der allgemeinen Formel (I), in denen n = 0 ist, wie in Formel (II) gezeigt.

Bevorzugt ist n = 0, 1 oder 2 und m = 3, 4 oder 5, weiter bevorzugt ist n = 0 oder 1 und m = 3 oder 4 und besonders bevorzugt ist n = 0 und m = 3 oder 4.

Bevorzugt ist n + m = 3, 4, 5 oder 6, besonders bevorzugt 3,4 oder 5 und ganz bevorzugt ist n + m = 3 oder 4, mit der Maßgabe, dass m ≥ 3 ist.

Bevorzugt ist n = 0, 1 oder 2 und m = 2, 3 oder 4 mit n + m = 3, 4, 5 oder 6, weiter bevorzugt ist n = 0 oder 1 und m = 2, 3 oder 4 mit n + m = 3, 4 oder 5 und besonders bevorzugt ist n = 0 und m = 3 oder 4 (n + m = 3 oder 4), jeweils mit der Maßgabe, dass m ≥ 3 ist.

Die erfindungsgemäßen Urethanmethacrylat-Verbindungen werden durch Umsetzen von einem Hydroxyalkylmethacrylat mit einem Diisocyanat und einem mindestens trifunktionellen Alkohol erhalten. Das Hydroxyalkylmethacrylat, das Diisocyanat und der Alkohol werden in Anwesenheit eines Katalysators und eines Inhibitors, welcher dazu dient, die gebildete Verbindung zu stabilisieren, zur Reaktion gebracht.

Geeignete Hydroxyalkylmethacrylate sind solche mit Alkylengruppen mit einem bis 15 Kohlenstoffatomen, wobei die Alkylengruppen linear oder verzweigt sein können. Bevorzugt sind Hydroxyalkylmethacrylate mit einem bis 10 Kohlenstoffatomen. Weiter bevorzugt sind Hydroxyalkylmethacrylate mit zwei bis sechs Kohlenstoffatomen, worunter 2-Hydroxyethylmethacrylat, 2-Hydroxypropylmethacrylat (2-HPMA), 3-Hydroxypropylmethacrylat (3-HPMA) und Glycerol-1,3-dimethacrylat besonders bevorzugt sind. Ganz besonders bevorzugt sind 2-Hydroxypropylmethacrylat (2-HPMA) oder 3-Hydroxypropylmethacrylat (3-HPMA).

Bevorzugte aromatische Diisocyanate sind solche mit aromatisch gebundenen Isocyanatgruppen, wie Diisocyanatobenzol, Toluoldiisocyanate (TDI), Diphenylmethandiisocyanate (MDI), Diisocyanatonaphthaline. Diese Verbindungen können sowohl als reine Verbindungen als auch optische Isomere oder Isomerengemische, in unterschiedlicher Zusammensetzung vorliegen, die gegebenenfalls in üblicher Art und Weise getrennt werden können.

Besonders bevorzugte aromatische Diisocyanate sind 1,4-Diisocyanatobenzol, 2,4-Diisocyanatotoluol, 2,6-Diisocyanatotoluol, 2,4'-Diphenylmethandiisocyanat, 4,4'-Diphenylmethandiisocyanat und 1,5-Diisocyanatonaphthalin.

Geeignete Alkohole sind tri- oder höherfunktionelle Alkohole, ausgewählt aus C₃-C₁₀-Alkoholen, bevorzugt C₃-C₄-Alkoholen mit den Hydroxylgruppen an den Enden und/oder entlang der Alkylkette. Beispiele für Alkohole mit mindestens drei OH-Gruppen umfassen Glycerin, Trimethylolmethan, Trimethylolethan, Trimethylolpropan, Trimethylolbutan, 1,2,4-Butantriol, 1,2,3-Hexantriol, 1,2,4-Hexantriol, Pentaerythrit, Diglycerin, Triglycerin, Bis(trimethylolpropan), Zucker, wie zum Beispiel Glucose, Zuckerderivate, wie z.B. Sorbit, Mannit, Diglycerol, Threit, Erythrit, Adonit (Ribit), Arabit (Lyxit), Xylit, Dulcit (Galactit), Maltit, Isomalt, oder Polyesterol, worunter Glycerin, Trimethylolpropan und Pentaerythrit bevorzugt sind.

Bevorzugt sind die erfindungsgemäßen Verbindungen der Formel (I) Verbindungen der allgemeinen Formel (la), (Ib), (IIa) oder (IIb): 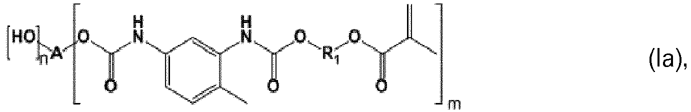 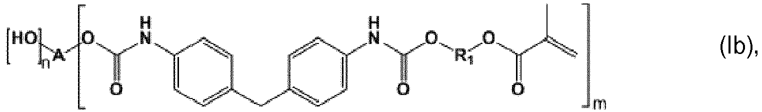 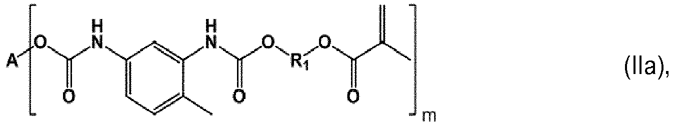 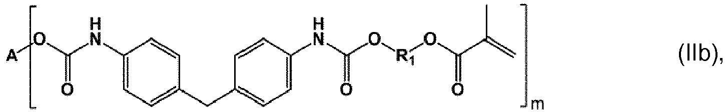 worin jeweils A, R₁, n und m wie oben für Formeln (I) und (II) definiert sind.

Besonders bevorzugte erfindungsgemäße Verbindungen sind Verbindungen der Formel (III) und (IV)

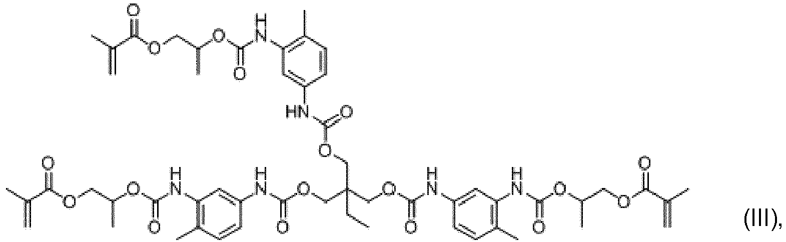

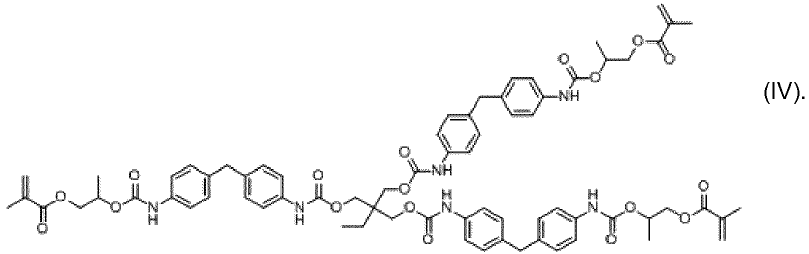

Die in den Formeln (I), (la), (Ib), (II), (IIa), (IIb), (III) und (IV) gezeigten Strukturen sollen die erfindungsgemäßen Verbindungen nur beispielhaft darstellen, da die zu deren Herstellung verwendeten Diisocyanate sowohl als isomerenreine Verbindungen als auch als Gemische der unterschiedlichen Isomere, in jeweils unterschiedlicher Zusammensetzung, d.h. in unterschiedlichen Mengenverhältnissen, eingesetzt werden können. Die gezeigten Strukturen sind daher nicht als einschränkend auszulegen.

Folglich können die erfindungsgemäßen Verbindungen als isomerenreine Verbindungen oder als Isomerengemische, in unterschiedlicher Zusammensetzung vorliegen, die gegebenenfalls in üblicher Art und Weise getrennt werden können. Sowohl die reinen Isomeren als auch die Isomerengemische sind Gegenstand der vorliegenden Erfindung. Gemische mit unterschiedlichen Anteilen an isomeren Verbindungen sind ebenfalls Gegenstand der Erfindung.

Ferner können je nach Reaktionsführung, insbesondere durch das Verhältnis von Isocyanatgruppen aus dem Diisocyanat zu Hydroxylgruppen aus dem Hydroxyalkylmethacrylat die Oligomere der Verbindungen der Formeln (I) bis (V) gebildet werden, so dass die Verbindungen eine Oligomerenverteilung haben. Gezeigt wird jeweils das Oligomer mit einer Wiederholungseinheit. Die Darstellung mit einer Wiederholungseinheit ist jedoch nicht einschränkend auszulegen, sondern dient der leichteren Darstellung. Die nicht gezeigten Oligomere, sofern diese vorhanden sind, werden auch von der Erfindung umfasst.

Für den Fall, dass bei der Herstellung der erfindungsgemäßen Verbindungen nicht alle Isocyanatgruppen umgesetzt werden oder ein Teil der Isocyanatgruppen vor der Reaktion etwa durch eine Nebenreaktion geöffnet werden, werden Verbindungen erhalten, die entweder als Hauptverbindungen oder als Verunreinigungen in den Reaktionsharz-Masterbatches enthalten sein können. Diese Verbindungen sind, sofern sie für die erfindungsgemäßen Zwecke verwendet werden können auch von der Erfindung umfasst.

Die Verbindungen der Formel (I) werden erfindungsgemäß zur Herstellung eines Reaktivharzes verwendet. Hierdurch kann eine Vernetzung der Polymerketten und damit die Ausbildung eines Polymernetzwerks erreicht werden. Dies kann sich positiv auf die Leistungsfähigkeit der ausgehärteten Masse auswirken.

Das erfindungsgemäße Reaktivharz enthält eine Verbindung der Formel (I) wie oben beschrieben als Backbone-Harz, einen Inhibitor, einen Beschleuniger und gegebenenfalls einen Reaktivverdünner. Da das Backbone-Harz nach seiner Herstellung typischerweise ohne Isolierung für die Herstellung des Reaktivharzes verwendet wird, sind in der Regel auch noch die neben dem Backbone-Harz im Reaktivharz-Masterbatch enthaltenen weiteren Bestandteile im Reaktivharz vorhanden, wie etwa einen Katalysator.

Die erfindungsgemäßen Verbindungen können dabei alleine oder zusätzlich zu anderen für den jeweiligen Anwendungszweck des Reaktivharzes üblicherweise verwendeten Harze verwendet werden. Damit kann die Vernetzungsdichte des Polymernetzwerks beeinflusst werden.

Der Anteil der Verbindung der allgemeinen Formel (I) in dem erfindungsgemäßen Reaktivharz beträgt von 25 Gew.-% bis 65 Gew.-%, vorzugsweise von 30 Gew.-% bis 45 Gew.-%, besonders bevorzugt von 35 Gew.-% bis 40 Gew.-%, ganz besonders bevorzugt von 33 Gew.-% bis 40 Gew.-% bezogen auf das Gesamtgewicht des Reaktivharzes.

Als Inhibitor sind die für radikalisch polymerisierbare Verbindungen üblicherweise verwendeten stabilen Radikale, wie *N*-Oxyl-Radikale geeignet, wie sie dem Fachmann bekannt sind.

Der Inhibitor kann zum einen dazu dienen, eine unerwünschte radikalische Polymerisation während der Synthese des Backbone-Harzes oder der Lagerung des Reaktivharzes und der Reaktivharzkomponente zu unterdrücken. Er kann auch dazu dienen - gegebenenfalls zusätzlich - die radikalische Polymerisation des Backbone-Harzes nach Zugabe des Initiators zeitlich zu verzögern und dadurch die Verarbeitungszeit des Reaktivharzes oder der Reaktivharzkomponente nach dem Mischen mit dem Härtungsmittel einzustellen.

Als stabile *N*-Oxyl-Radikale können beispielsweise solche verwendet werden, wie sie in der DE 199 56 509 A1 und der DE 195 31 649 A1 beschrieben sind. Derartige stabile

Nitroxylradikale sind vom Typ Piperidinyl-*N*-oxyl oder Tetrahydropyrrol-*N*-oxyl oder eine Mischung daraus.

Bevorzugte stabile Nitroxylradikale sind ausgewählt aus der Gruppe bestehend aus 1-Oxyl-2,2,6,6-tetramethylpiperidin, 1-Oxyl-2,2,6,6-tetramethylpiperidin-4-ol (ebenso als TEMPOL bezeichnet), 1-Oxyl-2,2,6,6-tetramethylpiperidin-4-on (ebenso als TEMPON bezeichnet), 1-Oxyl-2,2,6,6-tetramethyl-4-carboxyl-piperidin (ebenso als 4-Carboxy-TEMPO bezeichnet), 1-Oxyl-2,2,5,5-tetramethylpyrrolidin, 1-Oxyl-2,2,5,5-tetramethyl-3-carboxylpyrrolidin (ebenso als 3-Carboxy-PROXYL bezeichnet) und Mischungen aus zwei oder mehreren dieser Verbindungen, wobei 1-Oxyl-2,2,6,6-tetramethylpiperidin-4-ol (TEMPOL) besonders bevorzugt ist.

Neben dem Nitroxylradikal vom Typ Piperidinyl-*N*-oxyl oder Tetrahydropyrrol-*N*-oxyl können sowohl zur weiteren Stabilisierung des Reaktivharzes oder der das Reaktivharz enthaltenden Reaktivharzkomponente (A) oder anderer das Reaktivharz enthaltender Zusammensetzungen als auch zur Einstellung der Harzreaktivität ein oder mehrere weitere Inhibitoren vorhanden sein.

Hierfür sind die für radikalisch polymerisierbare Verbindungen üblicherweise verwendeten Inhibitoren geeignet, wie sie dem Fachmann bekannt sind. Bevorzugt sind diese weiteren Inhibitoren unter phenolischen Verbindungen und nicht-phenolischen Verbindungen und/oder Phenothiazinen, ausgewählt.

Als phenolische Inhibitoren, sind Phenole, wie 2-Methoxyphenol, 4-Methoxyphenol, 2,6-Di-tert-butyl-4-methylphenol, 2,4-Di-tert-butylphenol, 2,6-Di-tert-butylphenol, 2,4,6-Trimethylphenol, 2,4,6-Tris(dimethylaminomethyl)phenol, 4,4'-Thio-bis(3-methyl-6-tert-butylphenol), 4,4'-Isopropylidendiphenol, 6,6'-Di-tert-butyl-4,4'-bis(2,6-di-tert-butylphenol), 1,3,5-Trimethyl-2,4,6-tris(3,5-di-tert-butyl-4-hydroxybenzyl)benzol, 2,2'-Methylen-di-p-cresol, Catechole, wie Brenzkatechin, und Catecholderivate, wie Butylbrenzkatechine, wie 4-tert-Butylbrenzkatechin und 4,6-Di-tert-butylbrenzkatechin, Hydrochinone, wie Hydrochinon, 2-Methylhydrochinon, 2-tert-Butylhydrochinon, 2,5-Di-tert-butylhydrochinon, 2,6-Di-tert-butylhydrochinon, 2,6-Dimethylhydrochinon, 2,3,5-Trimethylhydrochinon, Benzochinon, 2,3,5,6-Tetrachloro-1,4-benzochinon, Methylbenzochinon, 2,6-Dimethylbenzochinon, Naphthochinon, oder Gemische von zweien oder mehreren davon, geeignet. Diese Inhibitoren sind oft Bestandteil von kommerziellen radikalisch härtenden Reaktivharzkomponenten.

Als nicht-phenolische Inhibitoren kommen vorzugsweise Phenothiazine, wie Phenothiazin und/oder Derivate oder Kombinationen davon, oder stabile organische Radikale, wie etwa Galvinoxyl- und *N-*oxyl-Radikale, jedoch nicht vom Piperidinyl-*N-*oxyl- oder Tetrahydropyrrol-*N-*oxyl-Typ, in Betracht, wie Aluminium-*N-*nitrosophenylhydroxylamin, Diethylhydroxylamin, Oxime, wie Acetaldoxim, Acetonoxim, Methylethylketoxim, Salicyloxim, Benzoxim, Glyoxime, Dimethylglyoxim, Aceton-O-(benzyloxycarbonyl)oxim und dergleichen.

Ferner können in para-Stellung zur Hydroxylgruppe substituierte Pyrimidinol- oder Pyridinol-Verbindungen, wie sie in der Patentschrift DE 10 2011 077 248 B1 beschrieben sind, als Inhibitoren eingesetzt werden.

Bevorzugt sind die weiteren Inhibitoren ausgewählt aus der Gruppe der Catechole, Catecholderivate, Phenothiazine, tert-Butylcatechol, Tempol oder ein Gemisch von Zwei oder mehr davon. Besonders bevorzugt sind die weiteren Inhibitoren ausgewählt aus der Gruppe der Catechole und Phenothiazine. Ganz besonders bevorzugt sind die in den Beispielen verwendeten weiteren Inhibitoren, bevorzugt etwa in den in den Beispielen angegebenen Mengen.

Die weiteren Inhibitoren können, abhängig von den gewünschten Eigenschaften des Reaktivharzes, entweder alleine oder als Kombination von zweien oder mehreren davon verwendet werden.

Der Inhibitor oder die Inhibitormischung wird zugesetzt in der Fachwelt bekannten üblichen Mengen, bevorzugt in einer Menge von etwa 0,0005 bis etwa 2 Gew.-% (bezogen auf das - letztendlich damit hergestellte - Reaktivharz), stärker bevorzugt von etwa 0,01 bis etwa 1 Gew.-% (bezogen auf das Reaktivharz), noch stärker bevorzugt von etwa 0,05 bis etwa 1 Gew.-% (bezogen auf das Reaktivharz), noch stärker bevorzugt von etwa 0,2 bis etwa 0,5 Gew.-% (bezogen auf das Reaktivharz).

Die Verbindungen der allgemeinen Formel (I), insbesondere bei der Verwendung in Reaktivharzen und Reaktivharzkomponenten für die chemische Befestigung und das bauliche Kleben, werden im Allgemeinen durch Peroxide als Härtungsmittel gehärtet. Die Peroxide werden bevorzugt durch einen Beschleuniger initiiert, damit auch bei niedrigen Anwendungstemperaturen eine Polymerisation stattfindet. Der Beschleuniger wird bereits dem Reaktivharz zugegeben.

Geeignete Beschleuniger, die dem Fachmann bekannt sind, sind beispielsweise Amine, bevorzugt tertiäre Amine und/oder Metallsalze.

Geeignete Amine sind unter folgenden Verbindungen ausgewählt: Dimethylamin, Trimethylamin, Ethylamin, Diethylamin, Triethylamin, n-Propylamin, Di-n-propylamin, Tri-n-propylamin, Isopropylamin, Diisopropylamin, Triisopropylamin, n-Butylamin, Isobutylamin, tert-Butylamin, Di-n-butylamin, Diisobutylamin, Tri-isobutylamin, Pentylamin, Isopentylamin, Diisopentylamin, Hexylamin, Octylamin, Dodecylamin, Laurylamin, Stearylamin, Aminoethanol, Diethanolamin, Triethanolamin, Aminohexanol, Ethoxyaminoethan, Dimethyl-(2-chloroethyl)amin, 2-Ethylhexylamin, Bis-(2-chloroethyl)amin, 2-Ethylhexylamin, Bis-(2-ethylhexyl)amin, N-Methylstearylamin, Dialkylamine, Ethylendiamin, N,N'-Dimethylethylendiamin, Tetramethylethylendiamin, Diethylentriamin, Permethyldiethylentriamin, Triethylentetramin, Tetraethylenpentamin, 1,2-Diaminopropan, Di-propylentriamin, Tripropylentetramin, 1,4-Diaminobutan, 1,6-Diaminohexan, 4-Amino-1-diethylaminopentan, 2,5-Diamino-2,5-dimethylhexan, Trimethylhexamethylendiamin, N, N-Dimethylaminoethanol, 2-(2-Diethylaminoethoxy)ethanol, Bis-(2-hydroxyethyl)-oleylamin, Tris-[2-(2-hydroxy-ethoxy)-ethyl]amin, 3-Amino-1-propanol, Methyl-(3-aminopropyl)ether, Ethyl-(3-aminopropyl)ether, 1,4-Butandiol-bis(3-aminopropylether), 3-Dimethylamino-1-propanol, 1-Amino-2-propanol, 1-Diethylamino-2-propanol, Diisopropanolamin, Methylbis-(2-hydroxypropyl)-amin, Tris-(2-hydroxypropyl)amin, 4-Amino-2-butanol, 2-Amino-2-methylpropanol, 2-Amino-2-methyl-propandiol, 2-Amino-2-hydroxymethylpropandiol, 5-Aiethylamino-2-pentanon, 3-Methylaminopropionsäurenitril, 6-Aminohexansäure, 11-Aminoundecansäure, 6-Aminohexansäureethylester, 11-Aminohexansäureisopropylester, Cyclohexylamin, N-Methylcyclohexylamin, N,N-Dimethylcyclohexylamin, Dicyclohexylamin, N-Ethylcyclohexylamin, N-(2-Hydroxyethyl)-cyclohexylamin, N,N-Bis-(2-hydroxyethyl)-cyclohexylamin, N-(3-Aminopropyl)-cyclohexylamin, Aminomethylcyclohexan, Hexahydrotoluidin, Hexahydrobenzylamin, Anilin, N-Methylanilin, N,N-Dimethylanilin, N,N-Diethylanilin, N,N-Di-propylanilin, iso-Butylanilin, Toluidine, Diphenylamin, Hydroxyethylanilin, Bis-(hydroxyethyl)anilin, Chloranilin, Aminophenole, Aminobenzoesäuren und deren Ester, Benzylamin, Dibenzylamin, Tribenzylamin, Methyldibenzylamin, a-Phenylethylamin, Xylidin, Diisopropylanilin, Dodecylanilin, Aminonaphthalin, N-Methylaminonaphthalin, N,N-Dimethylaminonaphthalin, N,N-Dibenzylnaphthalin, Diaminocyclohexan, 4,4'-Diaminodicyclohexylmethan, Diamino-dimethyl-dicyclohexylmethan, Phenylendiamin, Xylylendiamin, Diaminobiphenyl, Naphthalindiamine, Toluidine, Benzidine, 2,2-Bis-(aminophenyl)-propan, Aminoanisole, Amino-thiophenole, Aminodiphenylether, Aminocresole, Morpholin, N-Methylmorpholin, N-Phenylmorpholin, Hydroxyethylmorpholin, N-Methylpyrrolidin, Pyrrolidin, Piperidin, Hydroxyethylpiperidin, Pyrrole, Pyridine, Chinoline, Indole, Indolenine, Carbazole, Pyrazole, Imidazole, Thiazole, Pyrimidine, Chinoxaline, Aminomorpholin, Dimorpholinethan, [2,2,2]-Diazabicyclooctan und N,N-Dimethyl-p-toluidin.

Als Beschleuniger wird erfindungsgemäß Di-iso-propanol-p-toluidin oder N,N-Bis(2-hydroxyethyl)-m-toluidin eingesetzt.

Bevorzugte Amine sind Anilin-Derivate und N,N-Bisalkylarylamine, wie N,N,-Dimethylanilin, N,N-Diethylanilin, N,N-Dimethyl-p-toluidin, N,N-Bis(hydroxyalkyl)arylamine, N,N-Bis(2-hydroxyethyl)aniline, N,N-Bis(2-hydroxyethyl)toluidin, N,N-Bis(2-hydroxypropyl)anilin, N,N-Bis(2-hydroxypropyl)toluidin, N,N-Bis(3-methacryloyl-2-hydroxypropyl)-p-toluidin, N,N-Dibutoxyhydroxypropyl-p-toluidin und 4,4'-Bis(dimethylamino)diphenylmethan. Besonders bevorzugt ist Di-iso-propanol-p-toluidin.

Polymere Amine, wie solche die durch Polykondensation von N,N-Bis(hydroxylalkyl)anilin mit Dicarbonsäuren oder durch Polyaddition von Ethylenoxid oder anderen Epoxiden und diese Amine erhalten werden, sind ebenso als Beschleuniger geeignet.

Geeignete Metallsalze sind, zum Beispiel Cobaltoctoat oder Cobaltnaphthenoat sowie Vanadium-, Kalium-, Kalzium-, Kupfer-, Mangan- oder Zirkoniumcarboxylate. Weitere geeignete Metallsalze sind die weiter oben beschriebenen Zinn-Katalysatoren.

Sofern ein Beschleuniger verwendet wird, wird er in einer Menge von 0,01 bis 10 Gew.-%, bevorzugt 0,2 bis 5 Gew.-%, bezogen auf das Reaktivharz, eingesetzt.

Das Reaktivharz kann noch einen Reaktivverdünner enthalten, sofern dies erforderlich wird. Dabei kann ein gegebenenfalls bei der Herstellung des Backbone-Harzes eingesetzte Überschuss an hydroxyfunktionalisiertem (Meth)acrylat als Reaktivverdünner fungieren. Daneben, wenn das hydroxyfunktionalisierte (Meth)acrylat in etwa äquimolaren Mengen mit der Isocyanatgruppe eingesetzt wird, oder zusätzlich, falls ein Überschuss an hydroxyfunktionalisiertem (Meth)acrylat verwendet wird, können weitere Reaktivverdünner dem Reaktionsgemisch zugegeben werden, welche strukturell von dem hydroxyfunktionalisierten (Meth)acrylat verschieden sind.

Geeignete Reaktivverdünner sind niederviskose, radikalisch co-polymerisierbare Verbindungen, bevorzugt kennzeichnungsfreie Verbindungen, die zugegeben werden um unter anderem die Viskosität des Urethanmethacrylats bzw. der Vorstufen bei dessen Herstellung anzupassen, falls erforderlich.

Geeignete Reaktivverdünner sind in den Anmeldungen EP 1 935 860 A1 und DE 195 31 649 A1 beschrieben. Vorzugsweise enthält das Reaktivharz (die Harzmischung) als Reaktivverdünner einen (Meth)acrylsäureester, wobei besonders bevorzugt aliphatische oder aromatische C₅-C₁₅-(Meth)acrylate ausgewählt werden. Geeignete Beispiele umfassen: 2-Hydroxypropyl(meth)acrylat, 3-Hydroxypropyl(meth)acrylat, 1,2-Ethandioldi(meth)acrylat, 1,3-Propandioldimethacrylat, 1,3-Butandioldi(meth)acrylat, 1,4-Butandioldi(meth)acrylat, Trimethylolpropantri(meth)acrylat, Phenylethyl(meth)acrylat, Tetrahydrofurfuryl(meth)acrylat, Ethyltriglykol(meth)acrylat, *N,N-*Dimethylaminoethyl(meth)acrylat, N,N-Dimethylaminomethyl(meth)acrylat, N,N-Diethylaminoethyl(meth)acrylat, Acetoacetoxyethyl(meth)acrylat, Isobornyl(meth)acrylat, 2-Ethylhexyl(meth)acrylat, tert-Butylcyclohexyl(meth)acrylat, Benzyl(meth)acrylat, Methyl(meth)acrylat, n-Butyl(meth)acrylat, iso-Butyl(meth)acrylat, 3-Trimethoxysilylpropyl(meth)acrylat, Isodecyl(meth)acrylat, Diethylenglykoldi(meth)acrylat, Triethylenglykoldi(meth)acrylat, Methoxypolyethylenglykolmono(meth)acrylat, Trimethylcyclohexyl(meth)acrylat, 2-Hydroxyethyl(meth)acrylat, Dicyclopentenyloxyethyl(meth)acrylat und/oder Tricyclopentadienyldi(meth)acrylat, Bisphenol-A-(meth)acrylat, Novolakepoxidi(meth)acrylat, Di-[(meth)acryloyl-maleoyl]-tricyclo-5.2.1.0.2.6-decan, 3-(Meth)acryloyl-oxymethyl-tricylo-5.2.1.0.2.6-decan, 3-(Meth)cyclopentadienyl(meth)acrylat, und Decalyl-2-(meth)acrylat; PEG-di(meth)acrylat wie PEG200-di(meth)acrylat, Tetraehtylenglykoldi(meth)acrylat, Solketal(meth)acrylat, Cyclohexyl(meth)acrylat, Phenoxyethyldi(meth)acrylat, 2-Phenoxyethyl(meth)acrylat, Hexandiol-1,6-di(meth)acrylat, 1,2-Butandioldi(meth)acrylat, Methoxyethyl(meth)acrylat, Butyldiglykol(meth)acrylat, tert-Butyl(meth)acrylat und Norbornyl(meth)acrylat. Methacrylate sind gegenüber Acrylaten bevorzugt.

Besonders bevorzugt sind 2- und 3-Hydroxypropylmethacrylat, 1,2-Ethandioldimethacrylat, 1,4-Butandioldimethacrylat, 1,3-Butandioldimethacrylat, Glyceroldimethacrylat, Trimethylolpropantrimethacrylat, Acetoacetoxyethylmethacrylat, Isobornylmethacrylat, Bisphenol-A-dimethacrylat, ethoxylierte Bisphenol-A-methacrylate wie E2BADMA oder E3BADMA, Trimethylcyclohexylmethacrylat, 2-Hydroxyethylmethacrylat, PEG200-dimethacrylat und Norbornylmethacrylat und ganz besonders bevorzugt sind ein Gemisch aus 2- und 3-Hydroxypropylmethacrylat und 1,4-Butandioldimethacrylat oder eine Mischung aus diesen drei Methacrylaten.

Am bevorzugtesten ist ein Gemisch aus 2- und 3-Hydroxypropylmethacrylat. Grundsätzlich können auch andere übliche radikalisch polymerisierbare Verbindungen, allein oder im Gemisch mit den (Meth)acrylsäureestern, als Reaktivverdünner eingesetzt werden, z. B. Methacrylsäure, Styrol, α-Methylstyrol, alkylierte Styrole, wie tert-Butylstyrol, Divinylbenzol und Vinyl- sowie Allylverbindungen, wobei die nicht kennzeichnungspflichtigen Vertreter davon bevorzugt sind. Beispiele für derartige Vinyl- oder Allylverbindungen sind Hydroxybutylvinylether, Ethylenglycoldivinylether, 1,4-Butandioldivinylether, Trimethylolpropandivinylether, Trimethylolpropantrivinylether, Mono-, Di-, Tri-, Tetra- und Polyalkylenglycolvinylether, Mono-, Di-, Tri-, Tetra- und Polyalkylenglycolallylether, Adipinsäuredivinylester, Trimethylolpropandiallylether und Trimethylolpropantriallylether.

Der bzw. die Reaktivverdünner wird bzw. werden in einer Menge bis zu 65 Gew.-%, vorzugsweise bis zu 60 Gew.-%, weiter bevorzugt bis zu 55 Gew.-%, besonders bevorzugt in Mengen unter 50 Gew.-%, bezogen auf das Reaktivharz, zugegeben werden.

Ein beispielhaftes Reaktivharz umfasst eine Verbindung der allgemeinen Formel (I) 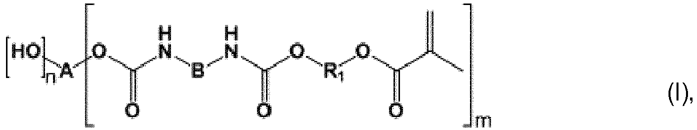 worin A, B, R₁, m und n wie oben definiert sind, als Backbone-Harz, ein stabiles Nitroxylradikal als Inhibitor, ein substituiertes Toluidin als Beschleuniger und gegebenenfalls einen Reaktivverdünner.

Ein bevorzugtes Reaktivharz umfasst eine Verbindung der Formel (II) 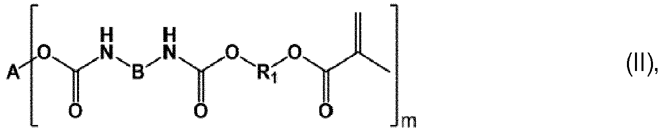 worin A, B, R₁ und m wie oben definiert ist, als Backbone-Harz, ein stabiles Nitroxylradikal als Inhibitor, ein substituiertes Toluidin als Beschleuniger und gegebenenfalls einen Reaktivverdünner.

Ein weiter bevorzugtes Reaktivharz umfasst eine Verbindung der Formel (III) oder (IV) 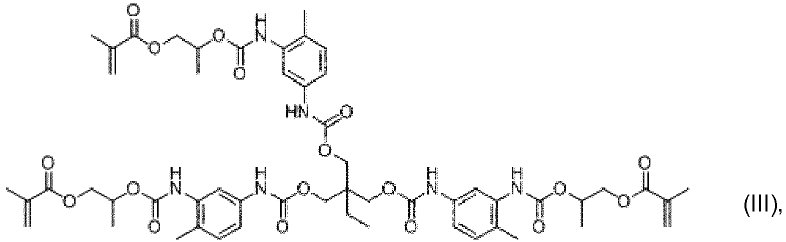 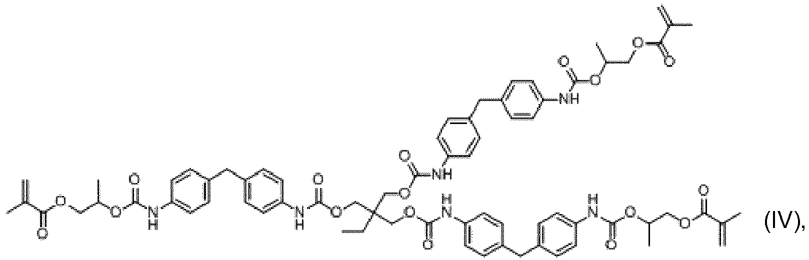 als Backbone-Harz, ein stabiles Nitroxylradikal als Inhibitor, ein substituiertes Toluidin als Beschleuniger und einen Reaktivverdünner.

Ein besonders bevorzugtes Reaktivharz umfasst eine Verbindung der Formel (III) oder (IV) als Backbone-Harz, 4-Hydroxy-2,2,6,6-tetramethyl-piperidinyl-1-oxyl (TEMPOL) als Inhibitor, Di-iso-propanol-p-toluidin als Beschleuniger und eine Mischung aus Hydroxypropylmethacrylat und 1,4-Butandioldimethacrylat (BDDMA) als Reaktivverdünner.

Ein erfindungsgemäßes Reaktivharz hat, aufgrund des verzweigten Backbone-Harzes, die Fähigkeit ein Polymernetzwerk zu bilden, so dass es möglich wird, eine Reaktivharzkomponente für ein Reaktivharz-System herzustellen, welche nach deren Aushärtung eine erhöhte Leistungsfähigkeit, insbesondere erhöhte Lastwerte zeigt als bei den herkömmlichen Systemen, ohne die dafür bisher benötigten Anteile an vernetzenden Reaktivverdünnern.

Ein weiterer Gegenstand der Erfindung ist eine Reaktivharzkomponente, welche das Reaktivharz enthält. Die Reaktivharzkomponente kann neben dem erfindungsgemäßen Reaktivharz anorganische Zuschlagstoffe, wie Füllstoffe und/oder Additive, enthalten. Es sei darauf hingewiesen, dass einige Stoffe sowohl als Füllstoff und, ggf. in modifizierter Form, auch als Additiv verwendet werden können. Beispielsweise dient pyrogene Kieselsäure in ihrer polaren, nicht nachbehandelten Form eher als Füllstoff und in ihrer apolaren, nachbehandelten Form eher als Additiv. In Fällen, in denen genau derselbe Stoff als Füllstoff oder Additiv verwendet werden kann, soll seine Gesamtmenge die hierin festgelegte Obergrenze für Füllstoffe nicht überschreiten.

Zur Herstellung einer Reaktivharzkomponente für bauliche Zwecke, inbesondere die chemische Befestigung können dem erfindungsgemäßen Reaktivharz übliche Füllstoffe und/oder Additive zugegeben werden. Diese Füllstoffe sind typischerweise anorganische Füllstoffe und Additive, wie beispielsweise weiter unten beschrieben.

Der Anteil des Reaktivharzes in der Reaktivharzkomponente beträgt bevorzugt von etwa 10 bis etwa 70 Gew.-%, stärker bevorzugt von etwa 30 bis etwa 50 Gew.-%, bezogen auf die Reaktivharzkomponente. Dementsprechend beträgt der Anteil der Füllstoffe bevorzugt von etwa 90 bis etwa 30 Gew.-%, stärker bevorzugt von etwa 70 bis etwa 50 Gew.-%, bezogen auf die Reaktivharzkomponente.

Als Füllstoffe finden übliche Füllstoffe, vorzugsweise mineralische oder mineralähnliche Füllstoffe, wie Quarz, Glas, Sand, Quarzsand, Quarzmehl, Porzellan, Korund, Keramik, Talkum, Kieselsäure (z.B. pyrogene Kieselsäure, insbesondere polare, nicht nachbehandelte pyrogene Kieselsäure), Silikate, Aluminiumoxide (z.B. Tonerde), Ton, Titandioxid, Kreide, Schwerspat, Feldspat, Basalt, Aluminiumhydroxid, Granit oder Sandstein, polymere Füllstoffe, wie Duroplaste, hydraulisch härtbare Füllstoffe, wie Gips, Branntkalk oder Zement (z.B. Aluminatzement (oft auch als Tonerdezement bezeichnet) oder Portlandzement), Metalle, wie Aluminium, Ruß, ferner Holz, mineralische oder organische Fasern, oder dergleichen, oder Gemische von zwei oder mehr davon Verwendung. Die Füllstoffe können in beliebigen Formen vorliegen, beispielsweise als Pulver oder Mehl, oder als Formkörper, z.B. in Zylinder-, Ring-, Kugel-, Plättchen-, Stäbchen-, Sattel- oder Kristallform, oder ferner in Faserform (fibrilläre Füllstoffe), und die entsprechenden Grundteilchen haben vorzugsweise einen maximalen Durchmesser von etwa 10 mm und einen Minimaldurchmesser von etwa 1 nm. Das heißt, der Durchmesser ist etwa 10 mm oder irgendein Wert von weniger als etwa 10 mm, aber mehr als etwa 1 nm. Bevorzugt ist der maximale Durchmesser ein Durchmesser von etwa 5 mm, bevorzugter von etwa 3 mm, noch bevorzugter von etwa 0,7 mm. Ganz besonders bevorzugt ist ein maximaler Durchmesser von etwa 0,5 mm. Der bevorzugtere Minimaldurchmesser ist etwa 10 nm, noch bevorzugter etwa 50 nm, ganz besonders bevorzugt etwa 100 nm. Durchmesserbereiche, die sich durch Kombination dieser maximalen Durchmesser und Minimaldurchmesser ergeben, sind besonders bevorzugt. Bevorzugt und deutlicher verstärkend wirken sich jedoch die globulären, inerten Stoffe (Kugelform) aus. Auch Core-Shell-Partikel, bevorzugt in Kugelform, können als Füllstoffe verwendet werden.

Bevorzugte Füllstoffe sind ausgewählt aus der Gruppe bestehend aus Zement, Kieselsäure, Quarz, Quarzsand, Quarzmehl, und Mischungen aus zwei oder mehreren davon. Für die Reaktivharzkomponente (A) besonders bevorzugt sind Füllstoffe ausgewählt aus der Gruppe bestehend aus Zement, pyrogener Kieselsäure, insbesondere unbehandelter, polarer pyrogener Kieselsäure, Quarzsand, Quarzmehl, und Mischungen aus zwei oder mehreren davon. Ganz besonders bevorzugt ist für die Reaktivharzkomponente (A) eine Mischung aus Zement (insbesondere Aluminatzement (oft auch als Tonerdezement bezeichnet) oder Portlandzement), pyrogener Kieselsäure und Quarzsand. Für die Härterkomponente (B) ist pyrogene Kieselsäure als alleiniger Füllstoff oder als einer von mehreren Füllstoffen bevorzugt; besonders bevorzugt sind neben der pyrogenen Kieselsäure noch einer oder mehrere weitere Füllstoffe vorhanden.

Als Additive finden übliche Additive Verwendung, also Thixotropiermittel, wie gegebenenfalls organisch oder anorganisch nachbehandelte pyrogene Kieselsäure (falls sie nicht schon als Füllstoff verwendet wird), insbesondere apolar nachbehandelte pyrogene Kieselsäure, Bentonite, Alkyl- und Methylcellulosen, Rhizinusölderivate oder dergleichen, Weichmacher, wie Phthalsäure- oder Sebacinsäureester, weitere Stabilisatoren neben den erfindungsgemäß verwendeten Stabilisatoren und Inhibitoren, Antistatikmittel, Verdickungsmittel, Flexibilisatoren, Rheologiehilfsmittel, Netzmittel, färbende Zusätze, wie Farbstoffe oder insbesondere Pigmente, beispielsweise zum unterschiedlichen Anfärben der Komponenten zur besseren Kontrolle von deren Durchmischung, oder dergleichen, oder Gemische von zwei oder mehr davon. Auch nicht reaktive Verdünnungsmittel (Lösungsmittel) können, vorzugsweise in einer Menge bis zu 30 Gew.-%, bezogen auf die Gesamtmenge der Reaktivharzkomponente enthalten sein, wie Niederalkylketone, z.B. Aceton, Diniederalkyl-niederalkanoylamide, wie Dimethylacetamid, Niederalkylbenzole, wie Xylole oder Toluol, Phthalsäureester oder Paraffine, Wasser oder Glykole. Ferner können Metallfänger in Form von oberflächenmodifizierten pyrogenen Kieselsäuren in der Reaktivharzkomponente enthalten sein. Bevorzugt ist mindestens ein Thixotropiermittel als Additiv vorhanden, besonders bevorzugt eine organisch oder anorganisch nachbehandelte pyrogene Kieselsäure, ganz besonders bevorzugt eine apolar nachbehandelte pyrogene Kieselsäure.

In dieser Hinsicht wird Bezug genommen auf die Anmeldungen WO 02/079341 und WO 02/079293 sowie WO 2011/128061 A1.

Der Anteil der Additive in der Reaktivharzkomponente kann bis zu etwa 5 Gew.-%, bezogen auf die Reaktivharzkomponente, betragen.

Die erfindungsgemäß hergestellten Reaktivharze sind in vielen Bereichen, bei denen sonst üblicherweise ungesättigte Polyesterharze, Vinylesterharze oder Vinylesterurethanharze Verwendung finden, einsetzbar. Sie finden üblicherweise Verwendung als Harzbestandteil in der Reaktivharzkomponente eines Reaktivharz-Systems, wie ein Mehrkomponenten-System, typischerweise ein Zweikomponenten-System aus einer Reaktivharzkomponente (A) und einer Härterkomponente (B). Dieses Mehrkomponenten-System kann in Form eines Patronen-Systems, eines Kartuschen-Systems oder eines Folienbeutel-Systems vorliegen. Bei der bestimmungsgemäßen Verwendung des Systems werden die Komponenten entweder unter Einwirkung mechanischer Kräfte oder durch Gasdruck aus den Patronen, Kartuschen oder Folienbeuteln ausgepresst, miteinander vermischt, vorzugsweise mit Hilfe eines Statikmischers, durch den die Bestandteile hindurchgeführt werden, und appliziert.

Gegenstand der vorliegenden Erfindung ist somit auch ein Reaktivharz-System mit einer eben beschriebenen Reaktivharzkomponente (A) und einer Härterkomponente (B), die einen Initiator für die Urethanmethacrylat-Verbindung enthält.

Der Initiator ist gewöhnlich ein Peroxid. Alle dem Fachmann bekannten Peroxide, die zum Härten von ungesättigten Polyesterharzen und Vinylesterharzen verwendet werden, können eingesetzt werden. Derartige Peroxide umfassen organische und anorganische Peroxide, entweder flüssig oder fest, wobei Wasserstoffperoxid auch verwendet werden kann. Beispiele geeigneter Peroxide sind Peroxycarbonate (der Formel -OC(O)O-), Peroxyester (der Formel -C(O)OO-), Diacylperoxide (der Formel -C(O)OOC(O)-), Dialkylperoxide (der Formel -OO-) und dergleichen. Diese können als Oligomer oder Polymer vorliegen.

Bevorzugt sind die Peroxide aus der Gruppe der organischen Peroxide ausgewählt. Geeignete organische Peroxide sind: tertiäre Alkylhydroperoxide, wie tert-Butylhydroperoxid, und andere Hydroperoxide, wie Cumenhydroperoxid, Peroxyester oder Persäuren, wie tert-Butylperester, Benzoylperoxid, Peracetate und Perbenzoate, Laurylperoxid, einschließlich (Di)peroxyesters, Perether, wie Peroxydiethylether, Perketone, wie Methylethylketoneperoxid. Die als Härter verwendeten organischen Peroxide sind oft tertiäre Perester oder tertiäre Hydroperoxide, d.h. PeroxidVerbindungen mit tertiären Kohlenstoffatomen, die direkt an eine -O-O-acyl- oder -OOH-Gruppe gebunden ist. Aber auch Gemische dieser Peroxide mit anderen Peroxiden können erfindungsgemäß eingesetzt werden. Die Peroxide können auch gemischte Peroxide sein, d.h. Peroxide, die zwei verschiedene Peroxid-tragende Einheiten in einem Molekül aufweisen. Bevorzugt wird zum Härten (Di-benzoyl)peroxid (BPO) verwendet.

Das Reaktivharz-System kann in Form eines Zwei- oder Mehrkomponenten-Systems vorliegen, bei dem die jeweiligen Komponenten räumlich getrennt voneinander vorliegen, so dass eine Reaktion (Aushärtung) der Komponenten erst nach deren Vermischen erfolgt.

Ein Zweikomponenten-Reaktivharz-System umfasst bevorzugt die A Komponente und die B Komponente reaktionsinhibierend getrennt in unterschiedlichen Behältern, beispielsweise einer Mehrkammer-Vorrichtung, wie eine Mehrkammer-Patrone und/oder -Kartusche, aus welchen Behältern die beiden Komponenten durch Einwirkung mechanischer Presskräfte oder unter Einwirkung eines Gasdruckes ausgepresst und vermischt werden. Eine weitere Möglichkeit besteht darin, das Zweikomponenten-Reaktivharzsystem als Zweikomponenten-Kapseln zu konfektionieren, die in das Bohrloch eingeführt werden und durch schlagdrehendes Setzen des Befestigungselements zerstört werden unter gleichzeitiger Durchmischung der beiden Komponenten der Mörtelmasse. Vorzugsweise wird hierin ein Patronensystem oder ein Injektionssystem eingesetzt, bei dem die beiden Komponenten aus den getrennten Behältern ausgepresst und durch einen statischen Mischer geführt werden, in dem sie homogen vermischt und dann über eine Düse vorzugsweise direkt in das Bohrloch ausgetragen werden.

Bei einer bevorzugten Ausführungsform des erfindungsgemäßen Reaktivharz-Systems ist das Reaktivharz-System ein Zweikomponenten-System und enthält die Reaktivharzkomponente (A) neben dem Backbone-Harz zusätzlich noch eine hydraulisch abbindende oder polykondensierbare anorganische Verbindung, insbesondere Zement, und die Härterkomponente (B) neben dem Initiator für die Polymerisation des Backbone-Harzes noch Wasser. Derartige Hybridmörtelsysteme sind ausführlich in DE 4231161 A1 beschrieben. Dabei enthält die Komponente (A) vorzugsweise als hydraulisch abbindende oder polykondensierbare anorganische Verbindung Zement, beispielsweise Portlandzement oder Tonerdezement, wobei übergangsmetalloxidfreie oder übergangsmetallarme Zemente besonders bevorzugt sind. Als hydraulisch abbindende anorganische Verbindung kann auch Gips als solcher oder in Mischung mit dem Zement eingesetzt werden. Die Komponente (A) kann als polykondensierbare anorganische Verbindung auch silikatische, polykondensierbare Verbindungen, insbesondere lösliches, gelöstes und/oder amorphes Siliziumdioxid enthaltende Stoffe wie z.B. polare, nicht nachbehandelte pyrogene Kieselsäure umfassen.

Das Volumenverhältnis von Komponente A zu Komponente B in einem Zweikomponenten-System ist vorzugsweise 3:1; 5:1 oder 7:1. Besonders bevorzugt ist ein Volumenverhältnis 3:1 oder 5:1.

In einer bevorzugten Ausführungsform enthält die Reaktivharzkomponente (A) also folglich:
- mindestens ein wie oben definiertes Urethan(meth)acrylat, bevorzugt eine Verbindung der Formel (IIa) oder (IIb);
- mindestens einen wie oben definierten Inhibitor vom Typ Piperidinyl-*N*-oxyl oder Tetrahydropyrrol-*N*-oxyl, bevorzugt TEMPOL;
- mindestens einen wie oben definierten Beschleuniger, bevorzugt ein Toluidin-Derivat, besonders bevorzugt Di-*iso*-propanol-*p*-toluidin;
- mindestens eine hydraulisch abbindende oder polykondensierbare anorganische Verbindung, bevorzugt Zement; und
- mindestens ein Thixotropiermittel, bevorzugt pyrogene Kieselsäure,
und die Härterkomponente (B) enthält:
- mindestens einen Initiator für die Initiierung der Polymerisation des Urethan(meth)acrylats, bevorzugt Benzoylperoxid (BPO) oder tert-Butylperoxybenzoat; und
- Wasser.

In einer bevorzugteren Ausführungsform enthält die Reaktivharzkomponente (A):
- mindestens ein wie oben definiertes Urethan(meth)acrylat, bevorzugt eine Verbindung der Formel (IIa) oder (IIb);
- mindestens einen wie oben definierten Inhibitor vom Typ Piperidinyl-*N*-oxyl oder Tetrahydropyrrol-*N*-oxyl, bevorzugt TEMPOL;
- mindestens einen Beschleuniger, bevorzugt ein Toluidin-Derivat, besonders bevorzugt Di-iso-propanol-p-toluidin;
- mindestens eine hydraulisch abbindende oder polykondensierbare anorganische Verbindung, bevorzugt Zement; und
- mindestens ein Thixotropiermittel, bevorzugt pyrogene Kieselsäure,
und die Härterkomponente (B) enthält:
- mindestens einen Initiator für die Initiierung der Polymerisation des Urethan(meth)acrylats, bevorzugt Benzoylperoxid (BPO) oder tert-Butylperoxybenzoat;
- mindestens einen Füllstoff, bevorzugt Quarzsand oder Quarzmehl; und
- Wasser.

In einer noch bevorzugteren Ausführungsform enthält die Reaktivharzkomponente (A):
- mindestens ein wie oben definiertes Urethan(meth)acrylat, bevorzugt eine Verbindung der Formel (IIa) oder (IIb);
- mindestens einen wie oben definierten Inhibitor vom Typ Piperidinyl-*N*-oxyl oder Tetrahydropyrrol-*N*-oxyl, bevorzugt TEMPOL;
- mindestens einen Beschleuniger, bevorzugt ein Toluidin-Derivat, besonders bevorzugt Di-*iso*-propanol-*p*-toluidin;
- mindestens einen weiteren Inhibitor, der ausgewählt ist aus der Gruppe besteht aus Catecholen und Phenothiazinen;
- mindestens eine hydraulisch abbindende oder polykondensierbare anorganische Verbindung, bevorzugt Zement; und
- mindestens ein Thixotropiermittel, bevorzugt pyrogene Kieselsäure,
und die Härterkomponente (B) enthält:
- mindestens einen Initiator für die Initiierung der Polymerisation des Urethan(meth)acrylats, bevorzugt Benzoylperoxid (BPO) oder tert-Butylperoxybenzoat;
- mindestens einen Füllstoff, bevorzugt Quarzsand oder Quarzmehl;
- mindestens ein Thixotropiermittel, bevorzugt pyrogene Kieselsäure; und
- Wasser.

In einer noch bevorzugteren Ausführungsform enthält die Reaktivharzkomponente (A):
- mindestens ein wie oben definiertes Urethan(meth)acrylat, bevorzugt eine Verbindung der Formel (III) oder (IV);
- mindestens einen wie oben definierten Inhibitor vom Typ Piperidinyl-*N*-oxyl oder Tetrahydropyrrol-*N*-oxyl, bevorzugt TEMPOL;
- mindestens einen Beschleuniger, bevorzugt ein Toluidin-Derivat, besonders bevorzugt Di-*iso*-propanol-*p*-toluidin;
- mindestens einen weiteren Inhibitor, der ausgewählt ist aus der Gruppe besteht aus Catecholen und Phenothiazinen;
- mindestens eine hydraulisch abbindende oder polykondensierbare anorganische Verbindung, bevorzugt Zement;
- mindestens ein Thixotropiermittel, bevorzugt pyrogene Kieselsäure, und
- mindestens einen weiteren Füllstoff, bevorzugt Quarzsand,
und die Härterkomponente (B) enthält:
- Benzoylperoxid (BPO) odertert-Butylperoxybenzoat als Initiatorfürdie Initiierung der Polymerisation des Urethan(meth)acrylats;
- mindestens einen Füllstoff, bevorzugt Quarzsand oder Quarzmehl;
- mindestens ein Thixotropiermittel, bevorzugt pyrogene Kieselsäure; und
- Wasser.

In einer noch bevorzugteren Ausführungsform enthält die Reaktivharzkomponente (A):
- mindestens ein wie oben definiertes Urethan(meth)acrylat, bevorzugt eine Verbindung der Formel (III) oder (IV);
- TEMPOL;
- Di-*iso*-propanol-*p*-toluidin;
- mindestens einen weiteren Inhibitor, der ausgewählt ist aus der Gruppe besteht aus Catecholen und Phenothiazinen;
- Zement;
- pyrogene Kieselsäure; und
- Quarzsand,
und die Härterkomponente (B) enthält:
- mindestens einen Initiator für die Initiierung der Polymerisation des Urethan(meth)acrylats;
- pyrogene Kieselsäure;
- Quarzsand oder Quarzmehl und
- Wasser.

In jeder dieser Ausführungsformen enthält in einer bevorzugten Ausführungsform die Reaktivharzkomponente (A) zusätzlich noch mindestens einen Reaktivverdünner.

Die Reaktivharzkomponenten (A) und die Härterkomponenten (B) in jeder dieser Ausführungsformen sind beliebig miteinander kombinierbar.

Ein solches Reaktivharz-System findet vor allem im Baubereich Verwendung (bauliche Zwecke), beispielsweise bei der Erstellung und Instandhaltung bzw. -setzung von Bauteilen und Bauwerken, etwa aus Beton, als Polymerbeton, als Beschichtungsmasse auf Kunstharzbasis oder als kalthärtende Straßenmarkierung, zur Verstärkung von Bauteilen und Bauwerken, beispielsweise Wände, Decken oder Böden, die Befestigung von Bauteilen, wie Platten oder Blöcken, z. B. aus Stein, Glas oder Kunststoff, an Bauteilen oder Bauwerken, etwa durch Kleben (bauliches Kleben). Besonders eignet sie sich zur chemischen Befestigung. Ganz besonders eignet sie sich zur (stoff- und/oder formschlüssigen) chemischen Befestigung von Verankerungsmitteln, wie Ankerstangen, Bolzen, Bewehrungseisen, Schrauben oder dergleichen in Aussparungen, wie Bohrlöchern, insbesondere in Bohrlöchern in verschiedenen Untergründen, insbesondere mineralischen Untergründen, wie solche auf der Grundlage von Beton, Porenbeton, Ziegelwerk, Kalksandstein, Sandstein, Naturstein, Glas und dergleichen, und metallischen Untergründen, wie solche aus Stahl. In einer Ausführungsform ist der Untergrund des Bohrlochs Beton, und das Verankerungsmittel besteht aus Stahl oder Eisen. In einer weiteren Ausführungsform ist der Untergrund des Bohrlochs Stahl, und das Verankerungsmittel besteht aus Stahl oder Eisen. Hierzu werden die Komponenten in das Bohrloch injiziert, wonach die zu befestigenden Einrichtungen, wie Ankergewindestangen und dergleichen, in das mit dem aushärtenden Reaktivharz beschickte Bohrloch eingebracht und entsprechend justiert werden.

Die folgenden Beispiele dienen der weiteren Erläuterung der Erfindung.

### BEISPIELE

Zunächst wurden Reaktivharz-Masterbatches, Reaktivharze, Reaktivharzkomponenten und Zweikomponenten-Reaktivharz-Systeme, die jeweils die erfindungsgemäße Verbindung (III) bzw. (IV) als Backbone-Harz enthalten, hergestellt. Die Verbundspannungen der ausgehärteten Befestigungsmassen wurden ermittelt.

### A1.1 Herstellung des Reaktivharz-Masterbatches A1.1 mit Verbindung (IV)

In einem 2 Liter Laborglasreaktor mit Innenthermometer und Rührwelle wurden 218 g Hydroxypropylmethacrylat und 669 g 1,4-Butandioldimethacrylat (BDDMA; Evonik AG) vorgelegt und mit 0,13 g Phenothiazin (D Prills; Allessa Chemie), 0,37 g 4-Hydroxy-2,2,6,6-tetramethyl-piperidinyl-1-oxyl (TEMPOL; Evonik Degussa GmbH), 0,23 g Dioctylzinndilaurat (TIB KAT^{®} 216; TIB Chemicals) und 67 g Trimethylolpropan (TMP) versetzt. Der Ansatz wurde auf 100°C erwärmt. Anschließend wurden 380 g Diphenylmethandiisocyanat (MDI; TCI Deutschland GmbH) (**3 Äquivalente pro Äquivalent TMP**) unter Rühren (200 U/min) über 70 Minuten zugetropft. Danach wurde weitere 300 Minuten bei 100°C gerührt. Abschließend wurden 666 g Hydroxypropylmethacrylat zugegeben.

Hierdurch wurde der Reaktivharz-Masterbatch A1.1 erhalten, der Verbindung (IV) als Backbone-Harz enthält. Das Produkt liegt als Oligomerverteilung vor, wobei das Oligomer mit einer Wiederholungseinheit die folgende Struktur hat:

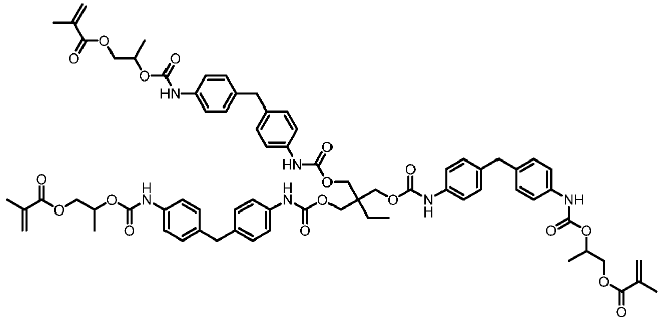

### A1.2 Herstellung des Reaktivharz-Masterbatches A1.2 mit Verbindung (IV)

In einem 2 Liter Laborglasreaktor mit Innenthermometer und Rührwelle wurden 300 g Hydroxypropylmethacrylat und 660 g 1,4-Butandioldimethacrylat (BDDMA; Evonik AG) vorgelegt und mit 0,12 g Phenothiazin (D Prills; Allessa Chemie), 0,29 g 4-Hydroxy-2,2,6,6-tetramethyl-piperidinyl-1-oxyl (TEMPOL; Evonik Degussa GmbH), 0,21 g Dioctylzinndilaurat (TIB KAT^{®} 216; TIB Chemicals) und 31 g Trimethylolpropan versetzt. Der Ansatz wurde auf 100°C erwärmt. Anschließend wurden 348 g MDI (**6 Äquivalente pro Äquivalent TMP**) unter Rühren (200 U/min) über 70 Minuten zugetropft. Danach wurde weitere 300 Minuten bei 100°C gerührt. Abschließend wurden 660 g Hydroxypropylmethacrylat zugegeben.

Hierdurch wurde der Reaktivharz-Masterbatch A1.2 erhalten, der die Verbindung (IV) als Backbone-Harz enthält. Das Produkt liegt ebenfalls als Oligomerverteilung vor, die jedoch anders ist als bei dem Produkt, das unter A1.1. hergestellt wurde.

### A2.1 Herstellung des Reaktivharzes A2.1

6,0 g 4-Hydroxy-2,2,6,6-tetramethyl-piperidinyl-1-oxyl (TEMPOL; Evonik Degussa GmbH) und 22,8 g Di-iso-propanol-p-toluidin (BASF SE) wurden zu 1271 g Reaktivharz-Masterbatch A1.1 gegeben.

Hierdurch wurde das Reaktivharz A2.1 erhalten, das die Verbindung (IV) als Backbone-Harz enthält.

### A2.2 Herstellung des Reaktivharzes A2.2

6,0 g 4-Hydroxy-2,2,6,6-tetramethyl-piperidinyl-1-oxyl (TEMPOL; Evonik Degussa GmbH) und 22,8 g Di-iso-propanol-p-toluidin (BASF SE) wurden zu 1271 g Reaktivharz-Masterbatch A1.2 gegeben.

Hierdurch wurde das Reaktivharz A2.2 erhalten, das die Verbindung (IV) als Backbone-Harz enthält.

Die Herstellung der Reaktivharze A2.1 und A2.2 erfolgte ohne die Zugabe weiterer Reaktivverdünner.

### A3.1 Herstellung der Reaktivharzkomponente A3.1

354 g Reaktivharz A2.1 wurden mit 185 g Secar^{®} 80 (Kerneos Inc.), 27 g Cab-O-Sil^{®} TS-720 (Cabot Corporation) und 335 g Quarzsand F32 (Quarzwerke GmbH) im Dissolver unter Vakuum mit einem PC Laborsystem Dissolver vom Typ LDV 0.3-1 vermischt. Die Mischung wurde für 8 Minuten bei 3500 U/min unter Vakuum (Druck≤100 mbar) mit einer 55 mm Dissolverscheibe und einem Randabstreifer gerührt.

Hierdurch wurde die Reaktivharzkomponente A3.1 erhalten.

### A3.2 Herstellung der Reaktivharzkomponente A3.2

354 g Reaktivharz A2.2 wurden mit 185 g Secar^{®} 80 (Kerneos Inc.), 27 g Cab-O-Sil^{®} TS-720 (Cabot Corporation) und 335 g Quarzsand F32 (Quarzwerke GmbH) im Dissolver unter Vakuum analog zu A3.1 vermischt.

Hierdurch wurde die Reaktivharzkomponente A3.2 erhalten.

### B1.1 Herstellung des Reaktivharz-Masterbatches B1.1 mit Verbindung (III)

In einem 2 Liter Laborglasreaktor mit Innenthermometer und Rührwelle wurden 271 g Hydroxypropylmethacrylat und 657 g 1,4-Butandioldimethacrylat (BDDMA; Evonik AG) vorgelegt und mit 0,16 g Phenothiazin (D Prills; Allessa Chemie), 0,39 g 4-Hydroxy-2,2,6,6-tetramethyl-piperidinyl-1-oxyl (TEMPOL; Evonik Degussa GmbH), 0,28 g Dioctylzinndilaurat (TIB KAT^{®} 216; TIB Chemicals) und 83 g Trimethylolpropan versetzt. Der Ansatz wurde auf 100°C erwärmt. Anschließend wurden 328 g Toluoldiisocyanat (TDI; TCI Deutschland AG) (**3 Äquivalente pro Äquivalent TMP**) unter Rühren (200 U/min) über 45 Minuten zugetropft. Danach wurde weitere 300 Minuten bei 100°C gerührt. Abschließend wurden 659 g Hydroxypropylmethacrylat zugegeben.

Hierdurch wurde der Reaktivharz-Masterbatch B1.1 erhalten, der die folgende Verbindung (III) als Backbone-Harz enthält. Das Produkt liegt als Oligomerverteilung vor, wobei das Oligomer mit einer Wiederholungseinheit die folgende Struktur hat:

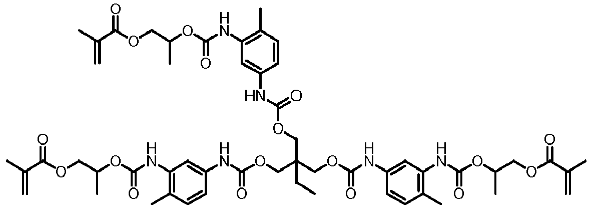

### B1.2 Herstellung des Reaktivharz-Masterbatches B1.2 Verbindung (III)

In einem 2 Liter Laborglasreaktor mit Innenthermometer und Rührwelle wurden 354 g Hydroxypropylmethacrylat und 660 g 1,4-Butandioldimethacrylat (BDDMA; Evonik AG) vorgelegt und mit 0,14 g Phenothiazin (D Prills; Allessa Chemie), 0,34 g 4-Hydroxy-2,2,6,6-tetramethyl-piperidinyl-1-oxyl (TEMPOL; Evonik Degussa GmbH), 0,24 g Dioctylzinndilaurat (TIB KAT^{®} 216; TIB Chemicals) und 37 g Trimethylolpropan versetzt. Der Ansatz wurde auf 100°C erwärmt. Anschließend wurden 286 g Toluoldiisocyanat (TDI; TCI Deutschland AG) (**6 Äquivalente pro Äquivalent TMP**) unter Rühren (200 U/min) über 45 Minuten zugetropft. Danach wurde weitere 300 Minuten bei 100°C gerührt. Abschließend wurden 662 g Hydroxypropylmethacrylat zugegeben.

Hierdurch wurde der Reaktivharz-Masterbatch B1.2 erhalten, der die Verbindung (III) als Backbone-Harz enthält. Das Produkt liegt ebenfalls als Oligomerverteilung vor, die anders ist als bei dem Produkt, das unter B1.1. hergestellt wurde.

### B2.1 Herstellung des Reaktivharzes B2.1

2,1 g 4-Hydroxy-2,2,6,6-tetramethyl-piperidinyl-1-oxyl (TEMPOL; Evonik Degussa GmbH) und 7,9 g Di-iso-propanol-p-toluidin (BASF SE) wurden zu 440 g Reaktivharz-Masterbatch B1.1 gegeben.

Hierdurch wurde das Reaktivharz B2.1 erhalten, das die Verbindung (III) als Backbone-Harz enthält.

### B2.2 Herstellung des Reaktivharzes B2.2

2,1 g 4-Hydroxy-2,2,6,6-tetramethyl-piperidinyl-1-oxyl (TEMPOL; Evonik Degussa GmbH) und 7,9 g Di-iso-propanol-p-toluidin (BASF SE) wurden zu 440 g Reaktivharz-Masterbatch B1.2 gegeben.

Hierdurch wurde das Reaktivharz B2.2 erhalten, das die Verbindung (III) als Backbone-Harz enthält.

Die Herstellung der Reaktivharze aus B2.1 und B2.2 erfolgte ebenfalls ohne die Zugabe weiterer Reaktivverdünner.

### B3.1 Herstellung der Reaktivharzkomponente B3.1

354 g Reaktivharz B2.1 wurden mit 185 g Secar^{®} 80 (Kerneos Inc.), 27 g Cab-O-Sil^{®} TS-720 (Cabot Corporation) und 335 g Quarzsand F32 (Quarzwerke) im Dissolver unter Vakuum analog zu A3.1 vermischt.

Hierdurch wurde die Reaktivharzkomponente B3.1 erhalten.

### B3.2 Herstellung der Reaktivharzkomponente B3.2

354 g Reaktivharz B2.2 wurden mit 185 g Secar^{®} 80 (Kerneos Inc.), 27 g Cab-O-Sil^{®} TS-720 (Cabot Corporation) und 335 g Quarzsand F32 (Quarzwerke GmbH) im Dissolver unter Vakuum analog zu A3.1 vermischt.

Hierdurch wurde die Reaktivharzkomponente B3.2 erhalten.

### C1. Herstellung des Vergleichsreaktivharz-Masterbatches C1 mit Vergleichsverbindung 1

Der Vergleichsreaktivharz-Masterbatch C1 mit 65 Gew.-% Vergleichsverbindung 1 als Backbone-Harz und 35 Gew.-% Hydroxypropylmethacrylat, bezogen auf das Gesamtgewicht des Vergleichsreaktivharz-Masterbatches, wurde gemäß dem Verfahren in EP 0 713 015 A1, die hiermit als Referenz eingeführt und auf deren gesamte Offenbarung verwiesen wird, hergestellt.

Das Produkt (Vergleichsverbindung 1) liegt als Oligomerverteilung vor, wobei das Oligomer mit einer Wiederholungseinheit die folgende Struktur hat:

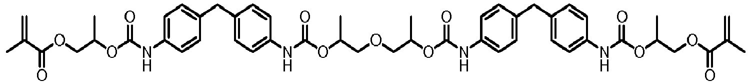

### C2. Herstellung des Vergleichsreaktivharzes C2

9,2 g 4-Hydroxy-2,2,6,6-tetramethyl-piperidinyl-1-oxyl (TEMPOL; Evonik Degussa GmbH) und 35,0 g Di-iso-propanol-p-toluidin (BASF SE) wurden zu einer Mischung aus 1004 g Vergleichsreaktivharz-Masterbatch C1, 300 g Hydroxypropylmethacrylat und 652 g 1,4-Butandioldimethacrylat (BDDMA; Evonik AG) gegeben.

Hierdurch wurde das Vergleichsreaktivharz C2 erhalten, das die Vergleichsverbindung 1 als Backbone-Harz enthält.

### C3. Herstellung der Vergleichsreaktivharzkomponente C3

354 g Vergleichsreaktivharz C2 wurden mit 185 g Secar^{®} 80 (Kerneos Inc.), 27 g Cab-O-Sil^{®} TS-720 (Cabot Corporation) und 335 g Quarzsand F32 (Quarzwerke GmbH) im Dissolver unter Vakuum analog zu A3.1 vermischt.

### Herstellung der Zweikomponenten-Reaktivharz-Systeme

Zur Herstellung der Zweikomponenten-Reaktivharz-Systeme A4.1, A4.2, B4,1 und B4.2 und des Vergleichszweikomponenten-Reaktivharz-Systems C4 wurden die Reaktivharzkomponenten A3.1, A3.2, B3.1 und B3.2 bzw. die Vergleichsreaktivharzkomponente C3 (Komponente (A)) und jeweils die Härterkomponente (Komponente (B)) des kommerziell erhältlichen Produkts HIT HY-110 (Hilti Aktiengesellschaft; Chargennummer: 1610264) in Plastikkartuschen (Firma Ritter GmbH; Volumenverhältnis A:B = 3:1) mit den Innendurchmessern 32,5 mm (Komponente (A)) bzw. 14 mm (Komponente (B)) gefüllt.

Hierdurch wurden die Zweikomponenten-Reaktivharz-Systeme A4.1, A4.2, B4.1 und B4.2 sowie das Vergleichszweikomponenten-Reaktivharz-System C4 erhalten.

Um den Einfluss der erfindungsgemäßen Verbindungen (III) und (IV) auf die Verbundspannungen einer erhärteten Befestigungsmasse zu zeigen, wurden die Verbundspannungen den ausgehärteten Befestigungsmassen, die die Reaktivharzkomponenten A3.1, A3.2, B3.1 und B3.2 enthalten, gemessen und mit der Verbundspannung der ausgehärteten Befestigungsmasse, die die Vergleichsreaktivharzkomponenten enthält, verglichen.

Zur Messung der Verbundspannungen (Lastwerte) der ausgehärteten Befestigungsmassen wurden Ankergewindestanden M12 in Bohrlöcher in Beton C20/25 mit einem Durchmesser von 14 mm und einer Bohrlochtiefe von 72 mm, die mit den Befestigungsmassen gefüllt wurden, eingeführt. Hierbei handelt es sich um gereinigte, staubfreie, trockene, hammergebohrte Bohrlöcher; die Aushärtung erfolgte bei 20°C. Die Temperatur des Zweikomponenten-Reaktivharz-Systems bzw. der Befestigungsmasse beträgt beim Setzen 20°C. Die Verbundspannungen wurden durch zentrisches Ausziehen der Ankergewindestanden ermittelt. Es wurden jeweils fünf Ankergewindestangen gesetzt und nach 24 Stunden Aushärtung die Verbundspannung bestimmt. Die Befestigungsmassen wurden über einen Statikmischer (Mischer HIT-RE-M; Hilti Aktiengesellschaft) aus den Kartuschen ausgepresst und in die Bohrlöcher injiziert.

Die hierbei ermittelten Verbundspannungen (N/mm²) sind als Mittelwert von fünf Messungen in der nachfolgenden Tabelle 1 aufgeführt.

**Tabelle 1: Ergebnisse der Messung der Verbundspannung der ausgehärteten Befestigungsmassen mit den Reaktivharzkomponenten A3.1, A3.2, B3.1 und B3.2 sowie der ausgehärteten Befestigungsmasse mit der Vergleichsreaktivharzkomponente C3**

| | **Verbundspannung [N/mm²]** |
|---|---|
| Befestigungsmasse mit Reaktivharzkomponente A3.1 | 21,3 |
| Befestigungsmasse mit Reaktivharzkomponente A3.2 | 19,4 |
| Befestigungsmasse mit Reaktivharzkomponente B3.1 | 20,0 |
| Befestigungsmasse mit Reaktivharzkomponente B3.2 | 18,2 |
| Vergleichsbefestigungsmasse mit Vergleichsreaktivharzkomponente C3 | 18,0 |

Die Ergebnisse zeigen, dass die Befestigungsmassen, welche mit den erfindungsgemäßen verzweigten Urethanmethacrylat-Verbindungen, Verbindung (III) und Verbindung (IV), hergestellt sind, höhere Verbundspannungen (Lastwerte) aufweisen als die Vergleichsbefestigungsmasse, die mit der Vergleichsverbindung 1 hergestellt wurde.

## Patentansprüche

1. Verbindung der allgemeinen Formel (I) 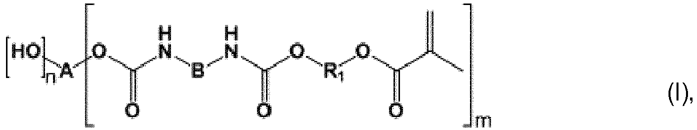 worin
B eine aromatische Kohlenwasserstoffgruppe ist,
A eine lineare oder verzweigte aliphatische C₃-C₁₀-Alkylengruppe ist,
jedes R₁ jeweils unabhängig voneinander eine verzweigte oder lineare aliphatische C₁-C₁₅-Alkylengruppe ist,
n eine ganze Zahl größer oder gleich 0 ist, und
m eine ganze Zahl größer oder gleich 3 ist.

2. Verbindung nach Anspruch 1, wobei B eine aromatische C₆-C₂₀-Kohlenstoffgruppe ist.

3. Verbindung nach Anspruch 2, wobei B einen oder zwei Benzolringe, die gegebenenfalls substituiert sind, enthält.

4. Verbindung nach einem der Ansprüche 1 bis 3, wobei R₁ eine C₂- oder C₃-Alkylengruppe ist.

5. Verbindung nach einem der vorhergehenden Ansprüche, wobei die lineare oder verzweigte aliphatische C₃-C₁₀-Alkylengruppe A eine drei- oder höherwertige, bevorzugt drei- oder vierwertige Gruppen ist, wie sie durch Entfernung der Hydroxylgruppen aus einem tri- oder höherfunktionellen, bevorzugt tri- oder tetrafunktionellen Alkohol erhalten wird.

6. Verbindung nach einem der vorhergehenden Ansprüche, wobei n = 0, 1 oder 2 ist und m = 3, 4 oder 5 ist

7. Verbindung nach Anspruch 6, wobei n = 0 oder 1 ist und m = 3, 4 oder 5 ist, mit n + m = 4 oder 5.

8. Verwendung Verbindung nach einem der vorhergehenden Ansprüche zur Herstellung eines Reaktivharzes oder einer Reaktivharzkomponente für bauliche Zwecke.

9. Verwendung einer Verbindung nach einem der Ansprüche 1 bis 7 zur Erhöhung der Verbundspannung einer ausgehärteten Befestigungsmasse.

10. Reaktivharz umfassend eine Verbindung nach einem der Ansprüche 1 bis 7, einen Inhibitor, einen Beschleuniger und gegebenenfalls einen Reaktivverdünner.

11. Reaktivharzkomponenten umfassend ein Reaktivharz nach Anspruch 10.

12. Reaktivharz-System mit einer Reaktivharzkomponente (A) nach Anspruch 11 und eine Härterkomponente (B), die einen Initiator enthält.

13. Reaktivharz-System nach Anspruch 12, wobei mindestens eine der Komponenten (A) oder (B) einen anorganischen Zuschlagstoff enthält.

14. Verwendung eines Reaktivharzes nach Anspruch 10, einer Reaktivharzkomponente nach Anspruch 11 oder eines Reaktivharz-Systems nach Anspruch 12 oder 13 für bauliche Zwecke.

15. Verwendung nach Anspruch 14 zur chemischen Befestigung von Verankerungsmitteln in Bohrlöchern.
